# EUROPEAN PATENT SPECIFICATION

(11) **EP 1 440 317 B1**
(45) Date of publication and mention of the grant of the patent: **20.06.2007**
(21) Application number: 02772626.4
(22) Date of filing: 04.11.2002
(51) Int. Cl.: G01N 33/58, G01J 3/44

(54) **MICROFLUIDIC SER(R)S DETECTION**
NACHWEIS DURCH SERRS IN MIKROFLUIDISCHER UMGEBUNG
DETECTION SERRS MICROFLUIDIQUE

(30) Priority: 02.11.2001 GB 0126319
(43) Date of publication of application: 28.07.2004
(73) Proprietor: University of Strathclyde, Glasgow G1 1XQ (GB); THE UNIVERSITY COURT OF THE UNIVERSITY OF GLASGOW, Glasgow, Lanarkshire G12 8QQ (GB)
(72) Inventor: SMITH, William, Ewen, Glasgow G61 3BD (GB); GRAHAM, Duncan, Edinburgh EH12 7LQ (GB); COOPER, Jonathan Mark, University of Glasgow, Glasgow G12 8QQ (GB); KEIR, Ruth, Perth PH2 0JJ (GB); IGATA, Eishi, Chiba 277-0005 (JP)
(74) Representative: Chapman, Paul Gilmour
(86) International application number: PCT/GB2002/005025
(87) International publication number: WO 2003/038436

(56) References cited:
- WO-A-01/31322
- WO-A-01/77650
- WO-A-99/18438
- WO-A-99/60157
- US-A- 6 025 202
- R. KEIR ET AL: "SERRS. In situ substrate formation and improved detection using microfluidics" ANALYTICAL CHEMISTRY, vol. 74, no. 7, 2002 - 1 April 2002 (2002-04-01), pages 1503-1508, XP002250986
- W. F. NIRODE ET AL: "On-column surface-enhanced Raman spectroscopy detection in capillary electrophoresis using running buffers containing silver colloidal solutions" ANALYTICAL CHEMISTRY, vol. 72, no. 8, 2000 - 15 April 2000 (2000-04-15), pages 1866-1871, XP002250987
- PATENT ABSTRACTS OF JAPAN vol. 2000, no. 22, 9 March 2001 (2001-03-09) & JP 2001 141655 A (MITSUBISHI HEAVY IND LTD), 25 May 2001 (2001-05-25)

### SEQUENCE LISTING

<110> UNIVERSITY OF STRATHCLYDE
<120> MICROFLUIDIC SERRS DETECTION
<130> PC/P12524PC
<140> PCT/GB02/005025
   <141> 2002-11-04
<150> GB0126319.3
   <151> 2001-11-02
<160> 1
<170> PatentIn version 3.1
<210> 1
   <211> 26
   <212> DNA
   <213> unknown
<220>
   <223> OLIGONUCLEOTIDE
<400> 1
   gtgctgcagg tgtaaacttg taccag 26

## Description

### Background to the invention

The present invention relates to a microfluidic method of generating *in situ* a colloid for use in detecting an analyte using for example SER (R) S, as well as a method of detecting an analyte using SER (R) S in a microfluidic system. The invention also relates to microfluidic devices for use in detecting analytes such as by way of SER(R)S signals.

Surface-enhanced resonance Raman scattering (1, 2) is an extremely powerful analytical tool which not only yields information about the molecular structure of the analyte in the form of a vibrational spectrum, but also allows sensitivity comparable to that achieved using fluorescence spectroscopy (3-5). Surface enhanced Raman scattering (SERS) involves the adsorption of an analyte on a suitable surface(usually roughened silver or gold) and the recording of the Raman scattering from that surface. The use of molecular resonance enhancement, where the frequency of the excitation source is tuned to be in resonance with an analyte chromophore as well as surface enhancement(SER(R)S), results in a further increase in sensitivity and also enhances the selectivity of the system(6). This enables discrimination of the analyte of interest from any contaminants which may be present in the system. Additionally, as compared to detection using fluorescence, there is much better labelling chemistry and much better results in multiplex detection.

One of the main problems associated with using SERRS as a quantitative analytical technique is the difficulty associated with producing reproducible SERRS substrates. Variations in the morphology of SERRS substrates can give rise to vast variations in the SERRS intensities produced(7). One common SERRS substrate is silver colloid(8-11). This can be easily prepared by reduction of aqueous solutions of silver salts(9, 12, 13). However, as with other substrates, there is still a problem associated with the production of stable, reproducible silver colloids(10, 14). It has also been found that in order to achieve the maximum enhancement from silver colloid the colloid must be aggregated(15-18). This is due to the greatly enhanced electromagnetic field between aggregated colloidal particles compared with that at the surface of a single colloidal particle(19). Aggregation can be induced using a wide range of reagents, however, this is a dynamic process and as different SERRS intensities are accumulated from aggregates of different morphologies(20, 21), it is desirable to be able to control the aggregation process in order to achieve reproducible SERRS signals.

One method of improving the reproducibility of SERRS signals obtained using silver colloid as the SERRS substrate, is to use a macro-flowcell for analysis(22-26). In this type of system the colloid, analyte and aggregating agent are pumped into a cell where they are allowed to mix. The signal is then accumulated from the flowing stream as it passes the laser beam. As the sample solution is continually flowing throughout the signal accumulation time, there is an averaging of the SERRS intensities accumulated from different aggregates and control over the kinetics of aggregate formation. This results in increased analytical precision and good quantitation over several orders of magnitude. This type of system also gives greater control over the rates of addition of reagents and the state of aggregation of the colloid at the point of signal accumulation. Similar systems have also been developed in which the colloid is prepared on-line(27-29). This eliminates the need to prepare stable, reproducible colloid batches.

However, there are a number of potential disadvantages with using macro-flow cell based systems. For example if colloid is prepared in *situ,* the colloid may only remain stable for a short period of time, typically minutes. This means essentially that the analyte sampling and SERRS detection have to be carried out concurrently. It is not generally possible therefore to take a sample and carry out the SERRS detection at a later time.

Moreover, with macro-flowcell systems a relatively large amount of reagents for carrying out the SERRS analysis is required, leading to impracticalities of field use of such devices. Additionally the sensitivity of SERRS detection using macro-flowcells may not be optimal due to an effective dilution of an analyte with the colloid. Other disadvantages of using a macro-flowcell system include the ability to store chemicals under non-degrading conditions and disposal of waste.

It is an object of the present invention to obviate and/or mitigate at least one of the aforementioned disadvantages.

### Summary of the Invention

In a first aspect the present invention provides method of generating *in situ* a colloid for use in detecting an analyte using SER(R)S, wherein the colloid is generated by contacting under conditions of laminar flow a first microfluidic stream of a suitable metal salt with a second microfluidic stream of a reducing agent whereby mixing of said first and second streams substantially occurs at an interface region between said first and second streams and wherein colloid is formed in the interface region and wherein the two streams are mixed in a channel of 1 µm to 500µm across.

The colloid so generated *in situ,* or ex situ (that is not using a micofluidic system, or a separate microfluidic system) may be mixed with an analyte in a microfluidic system having channels of 1 µm to 500µm across and under laminar flow conditions so as to adhere the analyte thereto and thereafter detected by way of SER(R)S. Optionally a further reagent may be bound or otherwise associated with the colloid and the analyte adhered thereto. Typically the analyte is provided by way of a third microfluidic stream arranged to contact the colloid so formed.

optionally an aggregating agent or means may also be required for colloid generation where the reducing agent alone is not sufficient for colloid generation. Said aggregating agent may be introduced by way of a further stream, or included in an existing stream which may be combined with said other streams, before, during, or after analyte mixing. Aggregating means may include electrochemical, electric, dielectric or magnetic means designed to aggregate the colloid. Such means could be positioned adjacent or in contact with the microfluidic stream, so as to act thereon.

It is to be appreciated that SER(R)S refers to SERS (surface enhanced Raman scattering) and SERRS (surface enhanced resonance Raman spectroscopy), with SERRS being preferred. This should not however be construed as limiting, as other scattering or wave-resonance detection techniques including Raleigh scattering and surface plasmon resonance could also be employed.

Examples of analytes which may be detected include, nucleic acids, nucleic acid analogues, proteins, peptides, amino acids, enzymes, prions, antibodies, aldehydes, amines, ketones, explosives, drugs of abuse, therapeutic agents, metabolites and environmental pollutants. This is not however exhaustive, as any suitable analyte may be detected. The analyte may be obtained from a sample and the sample may be any suitable preparation in which the target analyte is likely to be found. However, conveniently the sample may be in a fluid, or in solution or transferred to a solution before mixing with the colloid. Thus, for example when detecting explosives or drugs of abuse, a sample of gas, such as air or breath respectively, may be taken and any target analyte absorbed onto a suitable substrate. Thereafter, any target analyte may be removed from the substrate by washing with a suitable solvent.

For effective SERRS analysis, a chromophore of a suitable wavelength to be in resonance with the laser chosen, must be present in the analyte or a chromophore must be created by derivatisation of the analyte before analysis. Moreover in either case effective adsorption to the surface of the colloid particles must be achieved. Thus, the analyte may be reacted with a reagent so as to derivatise the analyte. The reagent which is used to derivatise the analyte, may provide a chromophore, may provide in combination with the analyte, a chromophore and/or render the analyte susceptible to adhering to the SERRS active substrate. Full details of such derivatisation may be found for example in PCT/GB01/01611, to which the skilled reader is directed.

The colloid particles prepared according to the present invention are aggregated in a controlled manner so as to be of a reproducible size and shape and as stable as possible against self-aggregation. Processes for preparing such unaggregated colloids are already known. They involve, for instance, the reduction of a metal salt (eg. silver nitrate) with a reducing agent such as citrate, to form a stable microcrystalline suspension (see P.C. Lee & D. Meisel, J. *Phys, Chem. (1982), 86, p3391).* This "stock" suspension is then aggregated *in situ* by contacting with a suitable aggregating agent. Suitable aggregating agents include acids (eg. HNO₃ or ascorbic acid), polyamines (eg. polylysine, spermine, spermidine, 1,4-diaminopiperazine, diethylenetriamine, N-(2-aminoethyl)-1,3-propanediamine, triethylenetetramine and tetraethylenepentamine) and inorganic activating ions such as Cl⁻, I⁻, Na⁺ or Mg²⁺. Heating may be required in certain circumstances to achieve colloid formation in a desirable timescale. A particularly preferred method of forming the colloid, which may be carried out at room temperature, is to mix a first stream comprising sodium-borohydride with a second stream comprising silver nitrate. To increase control over the process, all equipment used should be scrupulously clean, and reagents should be of a high grade. Unlike colloids prepared using existing techniques, the problem of precipitation does not occur. SER(R)S analysis may therefore be conducted for example 10 minutes to 4 hours after colloid aggregation, such as 15 minutes to 1 hour after aggregation.

The colloid particles are preferably monodisperse in nature and can be of any size so long as they give rise to a SERRS effect - generally they will be about 4 - 50 nm in diameter, preferably 25 - 36 nm, though this will depend on the type of metal. Any suitable metal or metal alloy may be used such as silver, copper or gold. Moreover, the particles may be coated on another surface, such as a bead or sphere, in order to, for example, increase the effective size/weight of the particles and thus alter their flow characteristics.

Preferably, the surface comprises metal, such as silver colloid particles, which may be substantially hexagonal in shape and of about 20 - 36 nm maximum diameter.

Adhering the analyte/derivatised analyte with the colloid will typically be by chemi-sorption of the complex onto the surface of the colloid particles, or by chemical bonding (covalent, chelating, etc.) of the complex with either the surface or a coating on the surface, either directly or through a linking group. The association will usually be via suitable functional groups on the analyte or derivatised analyte, such as charged polar groups (eg. NH₃⁺ or CO₂⁻), attracted to the surface or surface coating (eg. to free amine groups in a polyamine coating). Clearly, the type of association will depend on the nature of the surface and the label in any given case; different functional groups will be attracted to a positively-charged surface, for instance, as to a negatively-charged one.

Suitable groups by which the complex may be bound to the active surface include complexing groups such as nitrogen, oxygen, sulphur and phosphorous donors; chelating groups; bridging ligands and polymer forming ligands. Specific details of preferred methods of adhering the derivatised analyte with the SERRS active substrate are described, for example, in WO97/05280.

The method for obtaining the SERRS spectrum, once the derivatised analyte has been adhered to the metal substrate, may be conventional. By way of example, however, the following might apply to the spectroscopic measurements:

Typically, the methods of the invention will be carried out using incident light from a laser, having a frequency in or close to the visible spectrum ie. ^{~}380nm - 850nm, particularly between 400nm - 650nm (the exact frequency chosen will generally depend on the chromophore used in each case - frequencies in the red area of the visible spectrum tend, on the whole, to give rise to better surface enhancement effects but the fourth power of Raman means that blue is better and many chromophore lie in the green region giving maximum resonace enhancment there.. However, it is possible to envisage situations in which other frequencies, for instance in the ultraviolet (ie. 200nm - 400nm) or the near-infrared ranges (700nm - 1100nm), might be used. Thus, SERRS detection may be conducted between about 300nm - 1100nm.

The selection and, if necessary, tuning of an appropriate light source, with an appropriate frequency and power, will be well within the capabilities of one of ordinary skill in the art, particularly on referring to the available SERRS literature. To achieve highly sensitive detection, using SERRS, a coherent light source is needed with a frequency at or close to the absorption maximum for the chromophore (as described above) or that of the surface plasmons. If lower sensitivities are required, the light source need not be coherent or of high intensity and so lamps may be used in combination with a monochromator grating or prism to select an appropriate excitation frequency; here, there is no need to operate at the resonant frequency of the chromophore or the plasmons.

The light can be conducted from the source to the active surface by reflection in mirrors and can be focussed to give a higher light flux by passing through lenses. A suitable apparatus for SERRS analyses is a microscope with signal detection at 180 degrees to the excitation beam. A fluorescence microscope with confocal optics is also appropriate. The use of microscope optics permits the very small channels and/or volumes of a microfluidic device to be analysed.

Several devices are suitable for collecting SERRS signals, including wavelength selective mirrors, holographic optical elements for scattered light detection and fibre-optic waveguides. The intensity of a SERRS signal can be measured for example using a charge coupled device (CCD), a silicon photodiode, CMOS integrated detector, or photomultiplier tubes arranged either singly or in series for cascade amplification of the signal. Photon counting electronics can be used for sensitive detection. The choice of detector will largely depend on the sensitivity of detection required to carry out a particular assay.

Note that the methods of the invention may involve either obtaining a full SERRS spectrum across a range of wavelengths, or selecting a peak and scanning only at the wavelength of that peak (ie. Raman "imaging").

Apparatus for obtaining and/or analysing a SERRS spectrum will almost certainly include some form of data processor such as a computer.

Raman signals consist of a series of discrete spectral lines of varying intensity. The frequencies and the relative intensities of the lines are specific to the derivatised analyte being detected and the Raman signal is therefore a "fingerprint" of the derivatised analyte. If a SERRS analyser is being used selectively to detect one analyte out of a mixture then it will be necessary to detect the entire "fingerprint" spectrum for identification purposes. However if the analyser is being used to quantitate the detection of one or several analytes, each of which has a unique spectral line, then it will only be necessary to detect signal intensity at a chosen spectral line frequency or frequencies, or to detect all Raman scattering using a filter to exclude Rayleigh scattering.

Once the SERRS signal has been captured by an appropriate detector, its frequency and intensity data will typically be passed to a computer for analysis. Either the fingerprint Raman spectrum will be compared to reference spectra for identification of the detected Raman active compound or the signal intensity at the measured frequencies will be used to calculate the amount of Raman active compound detected.

A commercial SERRS analyser of use in carrying out the invention would be expected to consist of the following components: a laser light source, the appropriate optics for carrying the light to the SERRS active surface, a stage for mounting the microfluidic device for analysis, optics for receiving the Raman signal, a detector for converting the Raman signal into a series of intensities at certain wavelengths and a data processor for interpreting the wavelength/intensity data and providing an analytical output.

In a further aspect the present invention provides a microfluidic device for use in detecting analytes by way of SER(R)S, the device comprising a substrate having microscale channels of 1 µm to 500µm across formed therein and inlets attached to the channels for introducing a suitable metal salt, a reducing agent, a sample and optionally an aggregating agent, wherein laminar flow within channels for carrying the suitable metal salt and the reducing agent converge so as to allow mixing of the suitable metal salt and the reducing agent and generation of a colloid at an interface between the suitable metal salt flow and the reducing agent flow; and laminar flow within a channel for carrying the sample is arranged to converge with the colloid so produced such that any analyte present in the sample may adhere to the colloid.

The microfluidic device may be in the form of a Lab-on-a-chip device well known in the art and comprising all the necessary reagents within the chip. Alternatively the reagents can be provided from sources external to the device.

Typically the substrate is formed of glass, such as soda lime glass, for optical quality purposes although other clear materials may be used optically, such as silicon or polymers and the microscale channels have dimensions in the range 1µm to 500µm, typically 10µm - 200µm but most commonly 50µm. The size is however more dependent on being able to produce the appropriate laminar flow properties which have been observed as particularly beneficial. The inlets for introducing the various reagents are typically in the form of wells or reservoirs for maintaining a suitable quantity of reagent or sample.

Once any analyte has adhered to the colloid, detection of the analyte may be carried out by way of SER(R)S as hereinbefore described. Conveniently a microscope with a suitable lens may be focussed on a channel carrying colloid with adhered analyte.

The reagents and sample are drawn through the channels using suitable means, this may include for example, pumps, syringe drives, electrokinetics and/or electrohydrodynamics (see for example US 6,409,900).

Electroosmotic flow is an electrokinetic phenomenon that can be exploited to manipulate fluids within complex microfluidic manifolds. If silanol groups on the surface of a glass micro channel are deprotonated, positive ions from the bulk solution are attracted and are loosely bound to provide overall charge neutrality. When a high potential is applied across this channel, these loosely bound positive ions are attracted the cathode which through viscous coupling drags the bulk solution resulting in electroosmotic flow. One of the main advantages of EOF is the elimination of moving parts such as valves/switches. In addition, flat flow profiles are present eliminating the parabolic flow profile prevalent in pressure driven systems, i.e. the velocity is same across the whole channel. Typical channel dimensions used for successful EOF are approximately 10-100µm wide and 5-40µm deep with channel lengths in the regions of a few centimeters employing electrical field strengths of 100v-1kv/cm. Although electrophoretic forces are still present, the EOF is generally greater, allowing negative species such as a negative metal colloid or molecule to be driven towards the cathode.

In the case of colloid formation, aqueous silver salts can be mixed with an appropriate aqueous reducing agent such sodium borohydride to form a colloid stream. Exploiting the laminar flow properties of microfluidics, a stable and reproducible interface is formed between the two reagents at which reactions are dominated by diffusional processes rather than convective or mechanical means associated with macro scale formation. As such colloid size is more reproducible and precise. The use of such planar flow cells is compatible with optical microscopy regimes and is therefore well suited to SERRS detection, eliminating the problems commonly associated with focusing on circular capillaries/tubing

In microfluidic systems, low dimensions and fast fluid flow gives low Reynolds numbers and consequently no turbulence. Consequently, as two streams of fluid are brought together, liquid streamlines do not interfere with each other, and the only mixing that occurs is that provided by diffusion between the two streams. The process is known as laminar flow. The consequence of laminar flow and lack of mixing is often seen as a disadvantage for microfluidic systems. However, in the present invention the lack of bulk mixing has been utilised to provide the generation of colloid in a controlled manner.

In the context of detection by SER(R) S analysis, the production of colloidal metal, such as silver in a microfluidic system occurs through the bringing together of two streams of reactants. In such a case, silver nanoparticles are only produced at the interface between these two laminar streamlines, which do not, themselves mix.

The consequences of this are as follows:
(i) Increased Control of Colloidal Silver: The rate at which the colloid is produced (and hence the total amount of particulate silver) can be closely controlled and will be dependent upon the cross section of the microchannel, the concentration of reactants and the diffusion coefficients of the reactants. In particular, by maximising the height and minimising the width of the channel, for a given flow rate, concentration and cross sectional area, a greater amount of colloid will be produced. Moreover, a system can readily be optimised in which the amount of silver colloid production can be increased or decreased as a function of reaction conditions or microchannel geometry;
(ii) Localisation of the Colloid: The colloid is only produced at a given location, ie. at the interface between the laminar fluids. This has the effect of maximising the concentration of the colloid by reducing its dispersal. By reducing the analytical volume, there is the potential to increase the sensitivity of any measurement. In addition there is the further possibility of being able to isolate and further concentrate the colloid, due to its specific location; and
(iii)Retention of Colloid at a Specific Location: The colloid has a much lower diffusion coefficient than the reactants, such that once the colloid particles are in a given position, they diffuse away from that position at a greatly reduced rate (although the particles may still flow in the direction determined by the streamlines). This can improve sensitivity by enabling much greater accumulation times In addition, the colloid particles are sufficiently large that they can stabilise charge, encouraging electrostatic interactions with the substrate of the microfluidic channel, and thus enhancing the localisation of the particles. Microfluidic channels have a high surface to volume ratio, and so by definition, all the colloid will be close to the channel wall (an effect which is also dependent upon the geometry of the cross section of the channel).

Previously, some of the present inventors have demonstrated the integration of microelectrode arrays and/or waveguides or optical fibres in Lab-on-a-Chip devices (30). Using these microfabrication techniques, it is possible to further take advantage of the highly localised colloid particles of the present invention and achieve the following analytical advantages.
(i) Microelectrodes. The fabrication of a microelectrode array at a position proximal or within the channel can readily be achieved using photolithography, pattern transfer, metal deposition and lift-off. These electrode arrays can be used to further influence the interfacial stream of colloidal particles by either directing the stream into a separate analytical channel, chamber or region. Three such methods for doing this, all involving electric fields include either the use of electrophoresis or dielectrophoresis or the generation of an electro-osmotic flow. Further techniques which can be used to differentially move the colloid may involve the use fields generated by optical fields or by magnetism. In all cases, it will be possible to either separate the colloid from the reactants, and/or concentrate the colloid.
(ii) Waveguides/Fibres: It has also previously been demonstrated that microfabrication of optical circuits using either waveguides or fibres can be achieved (30). Such microfabricated devices may be used to further localise the excitation or collection of light. This has the effect of reducing the volume of the fluid being sampled, whilst increasing the sensitivity of the detection process. Such techniques would therefore lend themselves to detection of extremely rare biological events, including for example single molecule detection.

One application particularly suited to detection using the method and/or device according to the present invention is the detection of extremely small amounts of DNA for the analysis of single nucleotide polymorphisms (SNPs).

The basic approach makes use of the fact that SERRS can identify different chromophores based on molecular structure. Thus, a label can be generated in *situ* by the reaction of a SERRS activating agent and a specific tag attached to the molecule of interest. However, the tagged species does not produce any SERRS on its own, and only therefore produces a SERRS signal when in combination with the SERRS activating agent.

In one embodiment an oligonucleotide may be modified to contain the tag as described above. Oligonucleotides are used in a wide variety of analysis and one specific example is given below. The tag is a small unreactive chemical moiety that does not produce SERRS. i.e. no chromophores in the visible region and no propensity for the metal surface used. To generate a specific SERRS signal a chemical reaction that is specific to the tag (i.e. is not affected by any other chemical group) is used. This desirably has to be fast and occur in aqueous solution. One such reaction is a Diels Alder cycloaddition which actually occurs faster in water than organic solvent. The cycloaddition occurs between a diene and a dienophile. Thus it is possible to use either as the tag moiety. For the sake of brevity further reference will be made to the use of a diene as a tag on an oligonucleotide, but this is not to be construed as limiting. The tag may, for example, be added at the 5' or 3'-terminus or at any position within the oligonucleotide. For example, a furan residue which is a diene may be added to the 5'-terminus of an oligonucleotide and also to the 5-position of a thymidine nucleoside. The thymidine nucleoside can be used to add to any position of the probe oligonucleotide whilst being produced by solid phase synthesis.

A suitable dienophile is benzotriazole maleimide (BTM). The benzotriazole provides excellent surface attachment (see patent WO97/05280) and the maleimide is a dienophile commonly used in Diels Alder cycloadditions.

When the tagged oligonucleotide reacts with the BTM a unique signal is produced. Rather unexpectedly the quality of the signal is better than that of the pure, isolated cycloadduct without the attached oligonucleotide. Without wishing to be bound by theory this improvement is attributed to the presence of the oligonucleotide which confers increased aqueous solubility to the system.

A proposed method of DNA analysis using the method and/or device according to the present invention is as follows:

An object is to allow identification of single base polymorphisms within a gene based on the principle of mini-sequencing. A triphosphate with a diene tag may be produced according to the structures below.

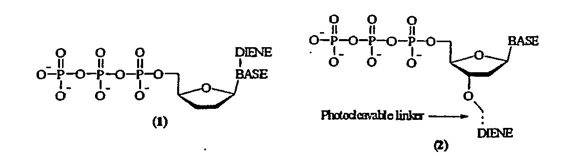

Thus, as the 3'-OH is missing (1) or blocked (2) only one base will be added in a round of enzymatic extension using a specific primer as is common in mini-sequencing. Once added, development takes place by addition of the dienophile to produce a unique SERRS active cycloadduct relating to the base added. In the case of triphosphate (2) the diene will be joined to the sugar via a photocleavable linkage. An example of a class of photocleavable linkers are nitrobenzyl groups and derivatives thereof, see (31). This will allow the diene to be removed to expose the 3'-OH again. This provides a primer that will hybridise as normal and a second round of sequencing can be performed to give the next base in the sequence. This may be done for example by recirculating the mix or by having a series of reaction chambers depending on the number of bases required for sequencing. The fact that the colloid stream remains in the middle of the channel and does not diffuse means that the tag can be removed photo chemically and will react with the developing reagent to give the SERRS active cycloadduct. This will actively move towards the colloidal stream and become adsorbed onto the surface of the colloid and is thus removed from further reaction. The liquid surrounding the colloid stream may then be recirculated and now contains a primer with one extra base added. This will allow the next base in the sequence to be identified by following the same procedure as for the first base.

The increased sensitivity of SERRS in a microflow cell means that this will be possible on DNA isolated directly from the organism without amplification. Advantages are that four compounds are all that's needed for identification of any number of mutations or normal bases. This approach cannot be done by fluorescence as fluorophores at the 3'-OH prevent the enzyme adding the triphosphate and fluorescence wouldn't give a fingerprint of the tag used.

Embodiments of the present invention relate to the ability to selectively code particles. For example it is possible to attach a number of different SER(R)S reactive dyes to the surface of a colloid particle as well as a reagent, such as a protein, peptide or oligonucleotide designed to bind to the analyte to be detected. This can be done either prior to introduction of the particle to the device or *in situ* in the dvice. By virtue of being able to effectively code the particles based on the particular dyes used, it is possible to detect analyte based on the particular SER(R)S signal generated. Complex mixtures of differently coded particles can be provided and readily identified based on the signal generated.

Also particles which have been functionalised with different dyes can be provided in a single system, whereby only one type of particle can bind to a particular analyte, thereby allowing detection of the analyte. An example of this would be to provide a number of separate oligonucleotides, each oligonucleotide being associated with a differently labeled particle, such that each oligonucleotide is capable of specifically binding to one form of a polymorphic nucleotide sequence, thereby enabling identification of the particular sequence in a sample being tested. Such a process is often referred to as multiplexing.

A final example relates to the ability to use the described method/system to introduce colloid particles into, for example, a biological cell, such as an animal cell. The cell can be introduced to a device so that the cell is immobilized or otherwise retaing the cell so that it is in the flow-path of the colloid. The cell can then be permeabilised by, for example, electroporation, such that colloid can flow into and/or through the cell. The colloid particles can be functionalised by attachment of, for example, an oligonucleotide, protein or peptide, which is designed to bind to a component within the cell. The exact location and/or presence of the component within the cell can then be detected by way of SER(R)S analysis.

The present invention will now be described by way of example and with reference to the Figures which show:
Figure la shows a schematic diagram of a chip design for use in generating colloid according to the present invention and detecting an analyte using SERRS;
Figure 1b shows an inset of a detail of a channel in the device as shown in Figure 1a;
Figure 1c shows a schematic diagram of a chip design according to the present invention, utilizing electroosmotic flow to manipulate fluids within the device;
Figure 2 shows the structure of 5-(2-methyl-3,5-dinitro-phenylazo)quinolin-8-ol;
Figure 3 shows an image of the point of colloid formation within the flow system channels, of the chip as shown in Figure 1a, collected using a white light microscope in reflection mode;
Figure 4 shows an image of the colloid stream as it passes through the part of the flow system, shown in Figure 1b, containing pillar structures, collected using a white light microscope in reflection mode;
Figure 5 shows images illustrating the diffusion of bromocresol purple (pH 9.0) indicator within the flow system at the point where the indicator meets the colloid stream, **a)** flowing **b)** to **e)** at increasing time intervals after flow has stopped;
Figure 6 shows images illustrating the diffusion of bromocresol indicator within the flow system at the point where there are pillar structures in the system, **a)** flowing **b)** to **e)** at increasing time intervals after flow has stopped;
Figure 7 shows images taken at the point of colloid formation **a)** immediately after colloid formation and **b)** 70 minutes after the flow had stopped. Both images were taken using a white light microscope in transmission mode;
Figure 8 shows images taken at the point in the system where there are pillared structures **a)** immediately after colloid formation and **b)** 70 minutes after the flow had stopped. Both images were taken using a white light microscope in transmission mode;
Figure 9 shows images taken at the point of colloid formation **a)** after colloid formation **b)** after distilled water has been passed through the system **c)** after cleaning with nitric acid and distilled water;
Figure 10 shows SERRS spectra of 5-(2-methyl-3,5-dinitro-phenylazo)quinolin-8-ol accumulated using the flow system. Concentration of dye within the flow system are **a)** 10 pico moles **b)** 1 pico mole **c)** 0.1 pico moles and **d)** 10 femto moles;
Figure 11 shows plot of SERRS intensity against time for signal accumulated from the same point under stopped flow conditions; and
Figure 12 a schematic diagram of a flow cell system for use in multiple mini-sequencing employing SERRS;
Figure 13 shows the separate and combined spectra of a laballed oligonucleotide and 3 additional dyes; and
Figures 14a and b show SER(R)S spectra obtained from 2 and 3 labeled oligonucleotides, respectively.

### Examples Section

### Example 1: Device Fabrication

The fluid channel was fabricated by a standard photolithographic technique. S1818 photoresist (Shipley Europe, Coventry, UK) was spin coated on an acid cleaned 1.5mm thick soda lime glass (Soda Lime glass, Nanofilm, USA) at 4000 rpm for 30 seconds. The glass was baked on a 90°C hot plate for 3 minutes, followed by UV exposure through an acetate sheet lithography mask to define the channel pattern for 8 seconds. The photoresist was developed in a mixed solution of 1 part of Microposit Developer Concentrate (Shipley Europe) and 1 part of RO water for 35 seconds and dried with nitrogen. The substrate was then baked on a 90°C hot plate for 15 minutes to ensure all the solvent had been evaporated and to harden the photoresist. Finally, the glass was wet etched in a mixed solution of 1 part of hydrofluoric acid and 4 parts of RO water for 15 minutes, resulting in 30 µm deep and 250 µm wide channel. After the etching procedure, the glass was ultrasonicated in acetone to remove the photoresist.

To seal the channels a cover plate was fabricated. Holes, for the inlet and outlet reservoirs, were aligned with the etched substrate and drilled using a 1.5mm diamond engraving bit (RS, Corby, Northants, UK). The etched substrate and cover plate were then prepared for bonding. Successful bonding relies on ultra clean surfaces which was carried out by cleaning the glass in a piranha solution (H₂SO₄ : H₂O₂ = 1 : 7) for at least 10 minutes, followed by a ultrasonication in acetone for 5 minutes. They were then rinsed in RO water and thoroughly dried with nitrogen. The etched substrate was then aligned with the access holes in the cover plate and placed in a steel clamp (Engineering Workshop, University of Glasgow) between two pieces of macor plates (RS, Corby, Northants, UK). The clamp was then placed in a furnace set at 60C which was ramped over 60 minutes to 500C and held for 1 hour. The temperature was then ramped up to 570C and held for a further 5 hours. Finally, the furnace was taken down to 60C and allowed to cool down overnight. The thermal bonding strategy resulted in permanent bond between the etched substrate and cover plate.

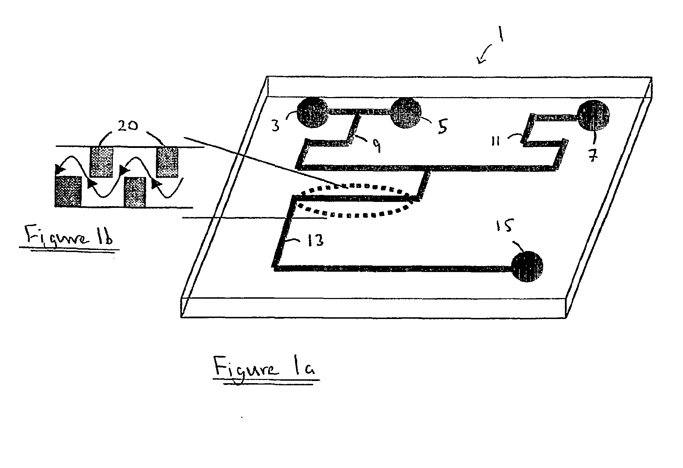

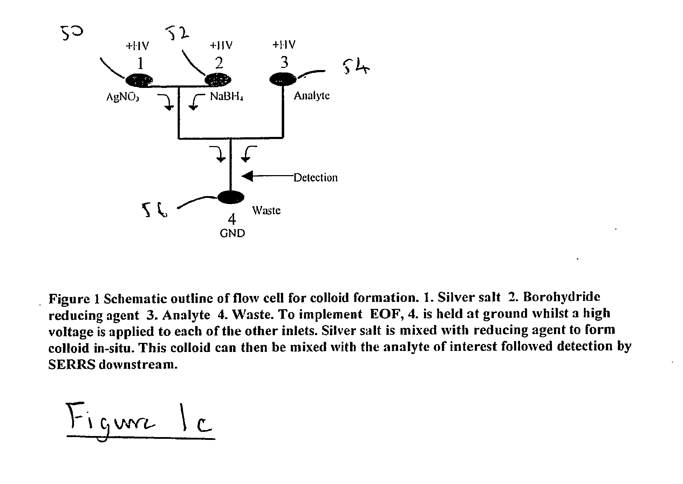

A diagram of the flow system used is shown in Figure 1a. Figure 1a shows a design of chip 1 suitable for use in the present invention. The chip 1 has inlets 3, 5 for introducing the reagents necessary for generating colloid and an inlet 7 for introducing a sample in which the analyte to be detected may be present. All solutions may be pulled through channels 9, 11, 13 by attaching a syringe to outlet 15. SERRS may be detected by focusing an appropriate laser anywhere along channel 13 ie. after colloid and analyte mixing.

Figure 1b is a diagram of a flow system designed to use elctroosmotic flow. Devices for colloid formation were fabricated from soda lime glass microscope slides. These devices were fabricated using standard photolithographic methods followed by hydrofluidic acid etching and thermal bonding of a blank glass coverplate. C dimension were 60µm wide, 10µm deep with a variety of lengths. The channel layout and mode of operation is shown figure 1b. Colloid forming reagents are introduced at wells, 50,52 and analyte at well 54. High voltages are applied to the device via platinum wire electrodes that are connected to a series of individual computer controlled high voltage power supplies (not shown) up to a maximum of 4Kv, whilst a waste outlet 56 is held at ground. The colloid formation and subsequent binding of analyte can be monitored down stream by SERRS.

### Example 2: Use of the Device and SERRS Detection

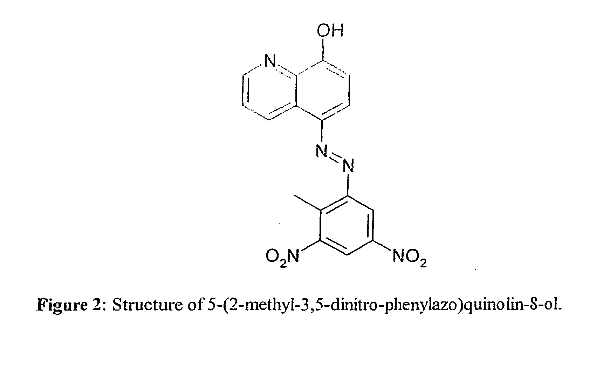

Sodium borohydride (99%), silver nitrate (99.9999%) and sodium hydroxide (97%) were purchased from Aldrich (Dorset, England). An aqueous solution of silver nitrate (2.6 x 10⁻³ M) and a solution of sodium borohydride (1.1 x 10⁻³ M) in sodium hydroxide solution (0.1 M), were introduced into inlets 3 and 5 respectively using a micropipette. The azo dye solution, which was prepared in methanol, was introduced into inlet 7. The chosen analyte was an azo dye, 5-(2-methyl-3,5-dinitrophenylazo)quinolin-8-ol, synthesised as part of a program to detect trinitrotoluene (TNT) by derivatisation methods. It was analysed by nmr and C,H and N analysis and found to be pure (C was within 0.4%). The structure of the dye is shown in Figure 2.

The solutions were pulled through the system under vacuum using a syringe attached to outlet 15. Spectra were accumulated by focussing the laser on the colloid stream using a x10 objective lens. Accumulation times were 10s. In the experiments involving pre-mixing of the dye solution with the borohydride solution, a mixture of 1:1 sodium borohydride and dye solution (1 x 10⁻⁶ M) was introduced into inlet 5, silver nitrate was introduced into inlet 3 and distilled water was introduced into inlet 7. The solutions were then pulled through the system using a syringe and spectra were accumulated using a Renishaw Mark I system 2000(Gloucs. England) with a Spectra Physics 361C 15mW argon ion laser working at 514nm, as the excitation source.

### Images

Images of the flow system were obtained using a white light microscope (Olympus) with a colour CCD camera (Polnex). Images were collected using a x5 microscope objective. The bromocresol purple solution (pH 9.0) was obtained from Micronics Inc.

### Results and discussion

### Flow Characteristics

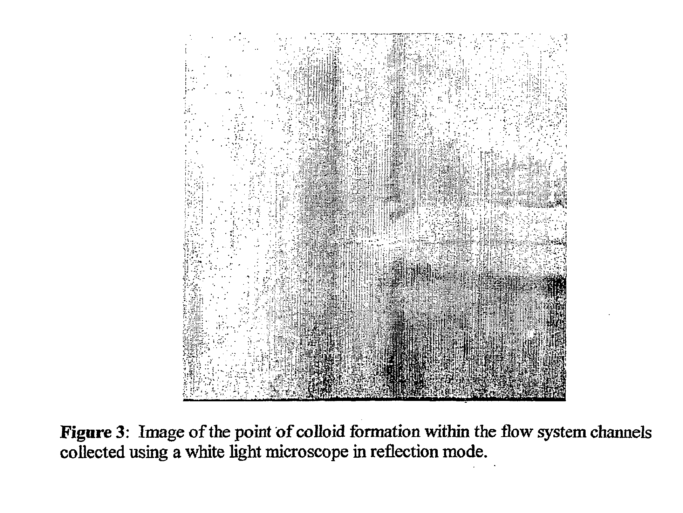

Introduction of silver nitrate and sodium borohydride solution into inlets 3 and 5 resulted in the formation of a thin line of colloid within the flow system channels. The colloid line could be followed through the system using a white light microscope in either transmission or reflection mode. An image of the point of colloid formation within the channel is shown in Figure 3. The diameter of the colloid line is approximately 30 µm, which is compatible with the use of a Raman microscope system, which can focus down to a 1-2 µm spot when using a x50 objective lens. This results in a SERRS system, where a large proportion of the stream is interrogated by the instrument as it flows past the interrogation point.

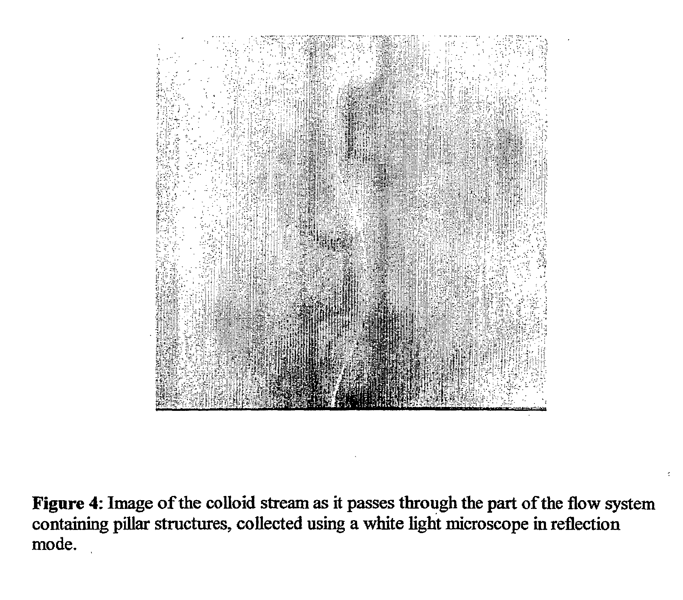

In order to try to promote mixing within the channels pillars 20 were introduced into the flow system. This is illustrated schematically in Figure 1b as an inset of the channel as shown in Figure 1a. By following the colloid flow through the system it could be seen that these features had no effect on the mixing of the colloidal particles. This is shown in Figure 4, which shows an image of the colloid stream as it passes through the part of the flowcell containing pillar structures.

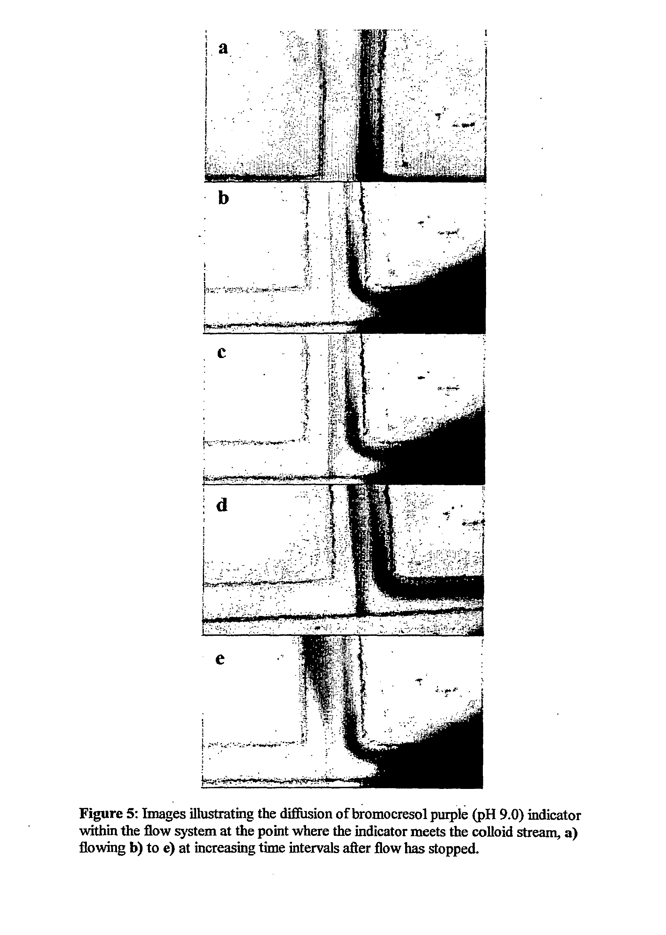

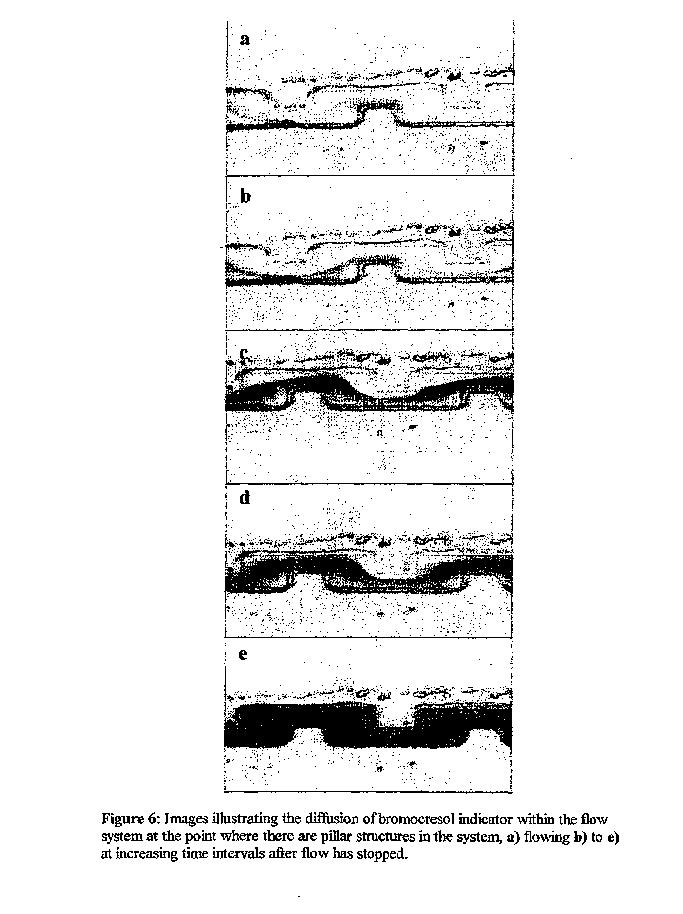

To illustrate the relative rates of mixing and diffusion of the colloidal particles and dye molecules within the flow system a solution of indicator, bromocresol purple (pH 9.0) was introduced into inlet 7. When this solution mixed with the sodium borohydride solution, which is in 0.1 M sodium hydroxide solution, the pH change caused the indicator colour to change from orange to purple. It was therefore possible to monitor the diffusion of the indicator molecules within the cell by recording the colour change. Again images were taken using the white light microscope, this time in transmission mode. Silver nitrate, sodium borohydride and bromocresol blue solutions were introduced into inlet 3,5 and 7 respectively and these solutions were pulled through the system using a syringe. The flow was then stopped and the solutions were allowed to diffuse within the channels. An image was taken while the solutions were flowing and at intervals over a period of approximately two seconds after the flow had stopped. These images are shown in Figure 5. It can be seen that there is no mixing of the indicator solution with the sodium hydroxide solution while the solutions are flowing. After the flow was stopped the solutions mixed to form a purple line in the middle of the channel at the point where the solutions met. Within approximately two seconds the indicator had diffused throughout the entire channel. Similar images were also collected at the point where there are pillar structures in the system. These images are shown in Figure 6. Here it can be seen that there is a fine purple line present in the centre of the channel when the solutions are flowing, perhaps indicating that the pillar structures do assist mixing of dye molecules within the channels. However, the appearance of the purple line during flow could be due to the fact that the pillars are further along the channels, therefore the solutions would have had more time to diffuse at this point. Also, pulling the solutions through the system using a syringe results in irreproducible flow rates and any variations in the amount of diffusion at different points in the system may be due to variations in the flow rate. Again it can be seen that within approximately two seconds the indicator had diffused throughout the channel. However, at both points in the system it can be seen that the colloid did not diffuse throughout the channel within the time taken for the indicator to diffuse. There are however differences in the thickness of the colloid line from one picture to the next in the sequences in Figures 5 and 6. It is thought that this may be due to a build up of silver on the glass surface of the flow system due to continual stopping and starting of the flow while the images were being collected.

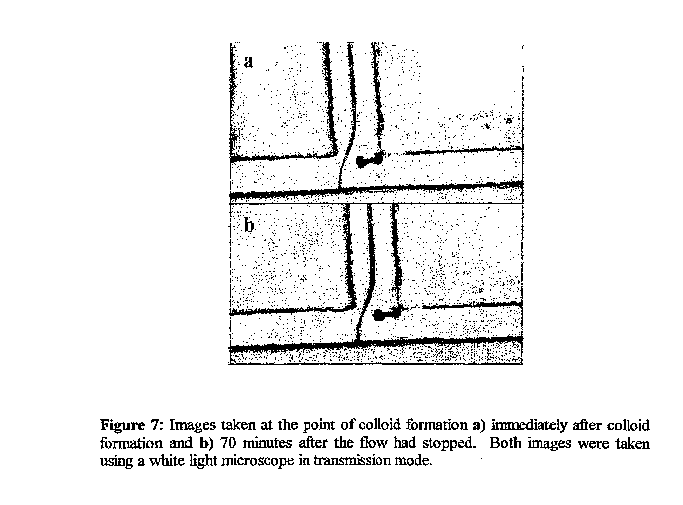

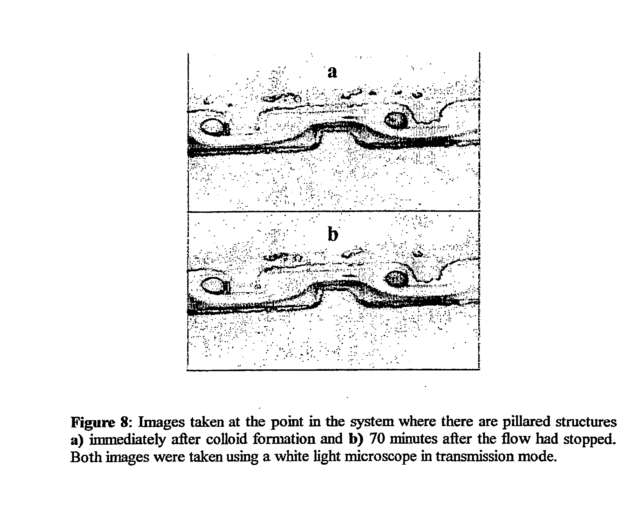

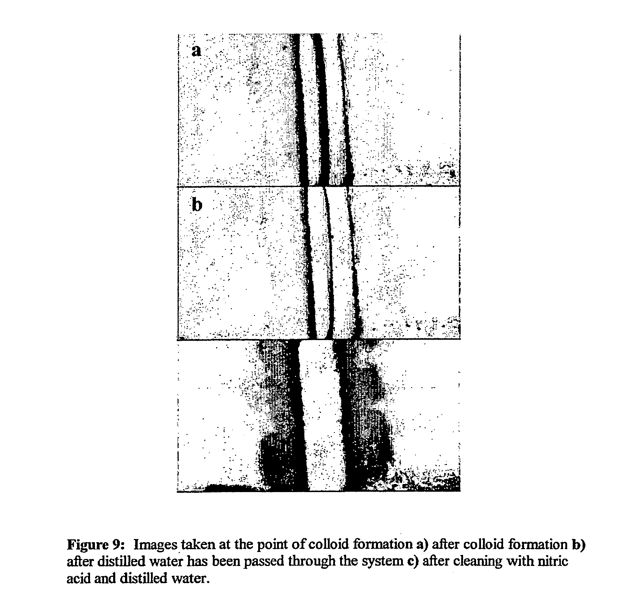

In order to show whether there is any colloid diffusion within the channels while the flow is stopped, silver nitrate, sodium borohydride and distilled water were introduced into inlets 3,5 and 7 respectively and these solutions were sucked through the flow system once. Images were then taken at the point of colloid formation, immediately after colloid formation and at various time intervals over a 70-minute period. The images taken immediately after colloid formation and after 70 minutes are shown in Figure 7. Here it can be seen that over the 70-minute period there is a slight darkening of the colloid stream suggesting that there is diffusion of the silver nitrate and sodium borohydride towards the centre of the channel where more colloid is formed. However the colloid stream itself does not disperse and no colloid is formed beyond the meeting point of the two solutions. The lack of diffusion of the colloidal particles may be due to the particles sticking to the walls of the flow system. The same experiment was carried out at the point in the flow system where there are pillared structures. The images taken immediately after colloid formation and after 70 minutes are shown in Figure 8. Here it can be seen that over the 70-minute period there was no change in the appearance of the colloid stream. The absence of any darkening with time suggested that all of the colloid has been formed by the time the stream had reached this part of the system. Again, there was no further diffusion of the colloid particles once the flow has stopped. The lack of diffusion may be caused by the charged colloidal particles attaching to the glass surface. In order to determine the extent of this attachment, colloid was formed in the system in the usual way and an image was taken after colloid formation. Distilled water was then pulled through the system and another image was taken. These images are shown in Figures 9a,b,c. It can be seen that some of the colloid is washed through the system by the distilled water, however, some does remain attached to the glass surface, which accounts partly for the lack of diffusion of the colloidal particles. Further washing with distilled water did not remove any more colloid from the glass surface. The remaining silver can be easily removed from the system by washing with nitric acid followed by distilled water. An image of the flow system after nitric acid cleaning can be seen in Figure 9c.

SERRS spectra of a dye 5-(2-methyl-3,5-dinitrophenylazo)quinolin-8-ol, were accumulated using this system by introducing silver nitrate, sodium borohydride and dye solution into inlets 3,5,7 respectively. In order to determine the sampling point from which the maximum signal intensity could be achieved, spectra were accumulated from various points throughout the system. Generally speaking the intensities of the spectra accumulated decreased after the point of colloid generation. This may be due to rapid colloid formation within the first half of system and a lack of diffusion of the colloidal particles throughout the remainder of the system. The higher concentration of silver in these parts of the system gives rise to an increase in the amount of silver surface available for dye attachment, therefore leading to an increase in signal intensity in these areas.

As it was found that there was an increase in the signal intensity towards the start of the chip, it was thought that premixing of the dye with either the sodium borohydride or silver nitrate solutions may lead to an increase in the signal intensity, as the dye would be present at the point in the system where the colloid is formed. In all cases when the dye was premixed with silver nitrate this resulted in no colloid formation. This was due to the dye complexing with the silver prior to mixing with sodium borohydride solution, therefore preventing colloid formation. Spectra were accumulated throughout the system by introducing silver nitrate into inlet 3 and a mixture of sodium borohydride and dye solution into inlet 5. The accumulation of spectra from the different points was randomised in order to eliminate any potential effects of the time of signal accumulation. As expected the most intense signals were accumulated from close to the point of colloid formation. There was however no significant difference between the signal intensities achieved from the point of colloid formation after premixing of the dye with sodium borohydride solution and those accumulated later in the system after introducing the dye through inlet 7.

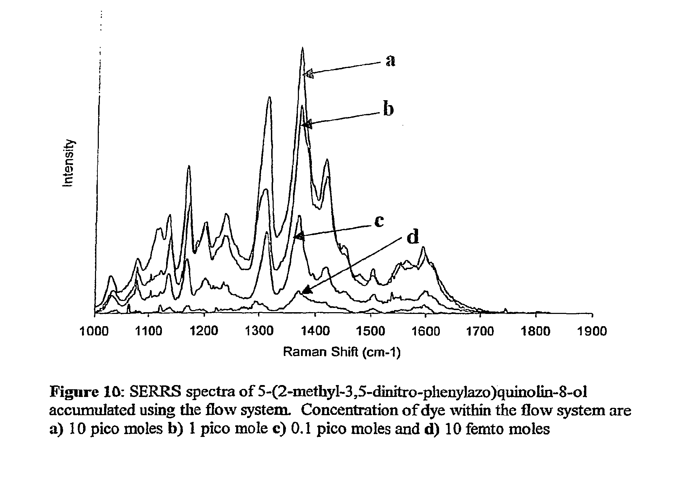

The absence of any diffusion of the colloid line once the flow has stopped makes this system suitable for analysis using a stopped flow system. This would enable accumulation of the SERRS signal over an extended period of time therefore decreasing the detection limits achievable using this system. In order to assess the potential for signal accumulation over an extended period of time, SERRS spectra of the dye were accumulated every minute for one hour from the same point in the system. A plot of the signal intensity against time is shown in Figure 10. It can be seen that the signal intensity remains constant for the first seven minutes before rapidly increasing. Without wishing to be bound by theory it is thought that this rapid increase in the signal intensity is due to aggregation of the colloidal stream due to continual exposure of the sample to the laser. It could be seen that the part of the colloidal stream where the signal was accumulated from had aggregated to form clusters of colloid. After this increase in signal, the signal decreases until at 50 minutes no signal can be seen due to burning of the sample. Although the signal does not remain constant throughout the 60 minute period, the a dye signal can be accumulated over a 50 minute period if necessary in order to decrease the potential detection limit.

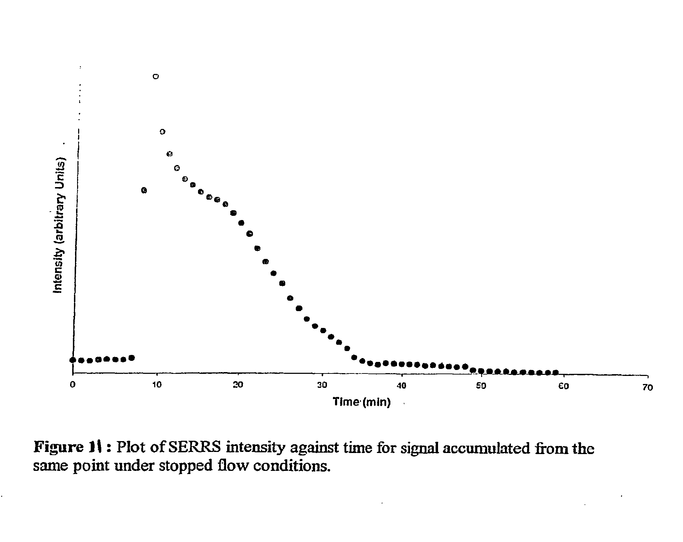

In order to determine the detection limit of the dye using this system, dye solutions of concentrations ranging from 10⁻⁶ M to 10⁻⁹ M were introduced into the system through inlet 3. The colloid was formed in the system as described above and 10 µL of each of the dye solutions was introduced into the system, resulting in dye concentrations ranging from 10 pico moles to 10 femto moles. The spectra were accumulated by focussing the laser on the colloid stream at the point where the dye solution met the colloid. The spectra accumulated are shown in Figure 11. Here it can be seen that it is possible to detect down to 10 femtomoles of the dye using this system. This represents a twenty-fold increase in sensitivity over that achieved using a macro-flowcell.

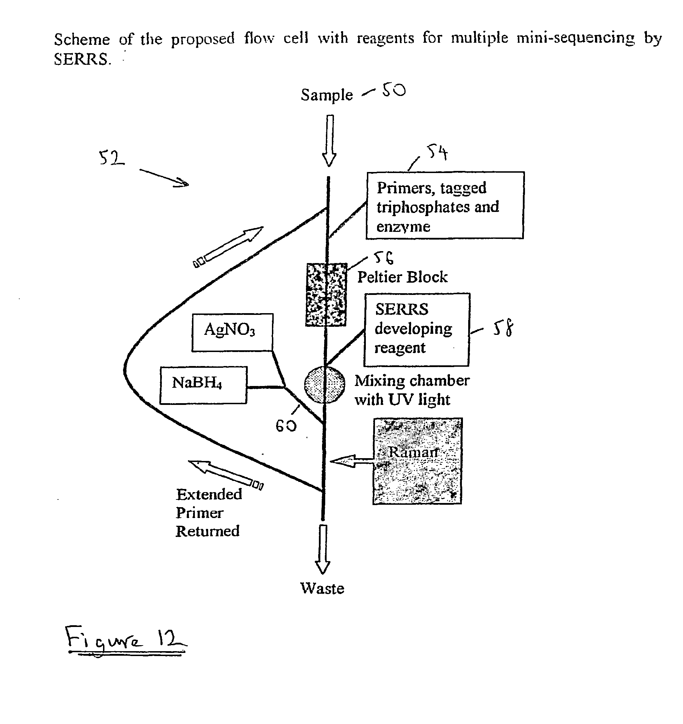

Figure 12 shows a schematic representation of a flow cell system according to the present invention which may be used in mini-sequencing to detect SNPs in a sample of DNA.

A sample of DNA 50 is introduced into the system 52 and thereafter allowed to mix with appropriate primers, tagged triphosphates and DNA polymerase 54. The triphosphates are tagged with a suitable diene as described earlier. Each triphosphate ie. ATP, CTP, GTP or TTP is tagged with a different diene, so that upon SERRS detection a signal corresponding to each particular diene is generated which may be equated with a particular nucleotide triphosphate. Extension of the primer by addition of a tagged triphosphate is achieved by passing the mixture comprising the sample of DNA 50, the primers, tagged triphosphates and DNA polymerase 54 over a Peltier block 56, the temperature of which is controlled to first denature the DNA and thereafter allow annealing of the primer and primer extension by enzymic addition of a tagged triphosphate. The tagged extended primer is then reacted with the SERRS developing reagent 58 such as benzotriazole maleimide. Thereafter the tagged diene and SERRS developing agent 58 are cleaved by use of UV light, leaving the extended primer ready for further reaction/extension before reacting with colloid 58 formed from AgNO₃ and NaBH₄ and the particular diene/SERRS developing agent detected by Raman spectroscopy. In this manner the identify of the added nucleotide triphosphate may be determined. The extended primer may be recycled for further reaction/extension and the waste removed.

### Example 3: Coded nanoparticles for detection of DNA by SERRS

Here it is shown that a labelled oligonucleotide can be detected with three other dyes in a suspension of nanoparticles indicating the ability to provide a coded nanoparticle for identification of a particular sequence in a mixture of DNA fragments.

The oligonucleotide examined contained a basic priming sequence of 5' GTG CTG CAG GTG TAA ACT TGT ACC AG 3'. The visible chromophore used was the fluorophore 2,5,2',4',5',7'-hexachloro-6-carboxyfluorescein (HEX), which was attached at the 5' terminus. HEX is negatively charged and therefore repels the negatively charged metal surface. Surface attachment was achieved by the incorporation of positively charged modified nucloebases at the 5'-terminus next to the HEX label. The three dyes used were azos containing the benzotriazole group for surface attachment.

All spectra were acquired in a Renishaw 2000 Raman Microprobe with a charge-coupled device (CCD) spectrometer. The excitation was provided by a Spectra-Physics Model 2020 argon-ion laser with a wavelength of 514.5nm and 2mW of power at the source. Samples were analysed in a plastic microtitre plate using a X50 objective. The acquisition time for all spectra was 10s and the grating was centred at 1350cm⁻¹.

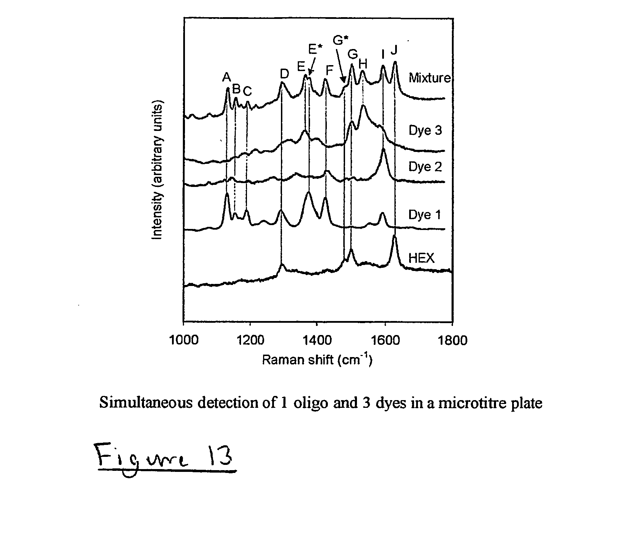

Figure 13 shows the spectra acquired from each of the individual dyes, including that with the oligonucleotide attached. For each sample 250µL of colloid, 250µL of distilled water, 30µL of analyte and 10µL of 0.067M spermine were mixed together and the SERRS was immediately acquired. Spermine is an effective aggregating agent for silver colloid. It is also a known charge neutralisation agent for the negatively charge phosphate backbone of DNA aiding adsorption of the sample to the metal surface. This figure also shows the spectrum recorded from the mixture of all dyes. All spectra have been normalised to have the same maximum intensity in the highest peak in each spectra.

### Example 4: Simultaneous detection of oligonucleotides without separation.

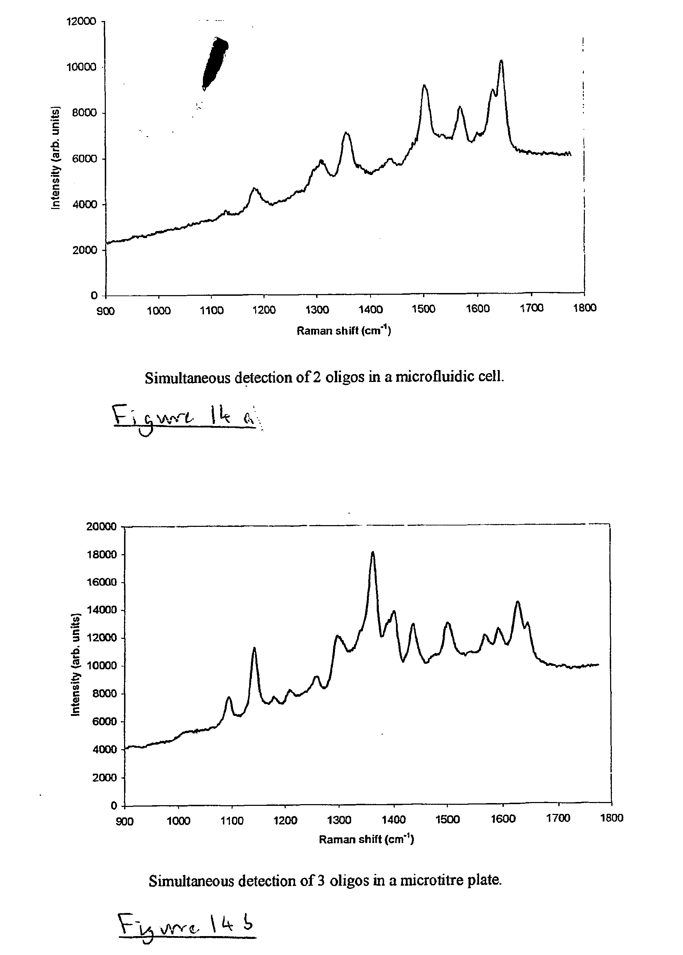

20µl of Rhodamine labelled oligonucleotide (17 mer), 20µl of Hex labelled oligonucleotide and 10µl of 0.067M spermine were premixed and added as a continuous flow into a microflow cell, as described in example 1. The cell was designed to enable the colloid to be produced in situ and the colloid and oligonucleotide stream was mixed. The Raman spectrum was collected shortly after the point of mixing in the cell for 5 seconds. The time between mixing and measurement was 20 seconds. Figure 14a clearly shows that signals from both oligonucleotides are easily distinguishable (01 rhodamine label, 02 Hex label).

Extension of this approach to three oligonucleotides is shown in figure 14b. In this case the oligonucleotides used were the Rhodamine and Hex labels and a third sequence labelled with an azo dye.

### Example 5: Electropermeabilization/electroporation of cells for inclusion of SERRS reactive particles

Motivation was to produce low resistance access to intracellular space in order to produce a reversible pore within the cell and to introduce SERRS particles. This has been achieved using both micron and submicron sized particles (sensor beads) enabling sensing to occur within the intracellular space.

Microelectrodes for electroporation were produced using standard photolithographic methods. Geometries were created to give a uniform fied across the cell or cells. Metal multilayers of Ti/Pd/Au (10/10/1000nm) were deposited against a photopaterned resist layer on a glass substrate and the geometry was realized by lift-off. A fluidic overlayer was defined in moulded PDMA and was sealed against the glass substrate. Dry etching of the glass prior to sealing gave a good fluidic seal, preventing leakage. The area for cell electroporation was prefunctionalised by either adsorption or attachment of an attachment motif or by polylysine 5mg/ml dried onto the surface. This functionalisation promoted cell adhesion, when introduced using a drawn micropipette, between the microelectrode geometries. The cell used was a rabbit cardiomyocyte prepared according to standard techniques.

Colloid was formed in the chip, as previously described under pressure driven flow, using two pumps, and the colloid flowed towards and round the cell. Two pairs of microelectrodes were used, one pair to electropermealise the cell, the second to monitor changes in local conductivity, such that it was possible to know that the cell compartment was accessed. The former pair of electrodes delivered a voltage pulse train of >200 mV for dielectric membrane breakdown. For example, ultrashort pulses (5 milliseconds) with 5-10 V DC enables reversible electropermeabilisation. Low voltage-pulses can be continually applied to a second orthogonal pair of electrodes to monitor the conductance of the cell, and hence the extent of permeabilisation. Groups of colloidal particles were viewed to have entered the cell, by visual observation of the cell under an inverted microscope.

In the preferred format, Au particles were used as they were found to be less toxic.

### REFERENCES

(1) Moskovits, M. J. Chem. Phys. 1978, 69, 4159-4161.
(2) Fleischmann, M.; Hendra, P.J.; McQuillan, A.J. J. Chem. Phys. Lett , 26, 163-166.
(3) Nie, S.M.; Emery, S.R. Science 1997, 275, 1102-1106.
(4) Kneipp, K.; Wang, Y.; Dasari, R.R.; Feld, M.S. Applied Spectroscopy 1995, 49, 780-784.
(5) Rodger, C.; Smith, W.E.;. Dent, G.; Edmondson, M. Journal of the Chemical Society-Dalton Transactions 1996, 791-799.
(6) Hildebrandt, P.; Stockburger, M. Journal of Physical Chemistry 1984, 88, 5935-5944.
(7) Yang, X.M.; Ajito, K.; Tryk, D.A.; Hashimoto, K.; Fujishima, A. Journal of Physical Chemistry 1996, 100, 7293-7297.
(8) Laserna, J.J.; Torres, E.L.; Winefordner, J.D. Analytica Chimica Acta 1987, 200, 469-480.
(9) Cermakova, K.; Sestak, O.; Matejka, P.; Baumruk, V.; Vlckova, B. Collection of Czechoslovak Chemical Communications 1993, 58, 2682-2694.
(10) Schneider, S.; Halbig, P.; Grau, H.; Nickel, U. Photochemistry and Photobiology 1994, 60, 605-610.
(11) Vlckova, B.; SoleckaCermakova, K.; Matejka, P.; Baumruk, V. Journal of Molecular Structure 1997, 408, 149-154.
(12) Lee, P.C.; Meisel, D. Journal of Physical Chemistry 1982, 86, 3391-3395.
(13) Munro, C.H.; Smith, W.E.; Garner, M.; Clarkson, J.; White, P.C. Langmuir 1995, 11, 3712-3720.
(14) Shirtcliffe, N.; Nickel, U.; Schneider, S. Journal of Colloid and Interface Science 1999, 211, 122-129.
(15) Campbell, M.; Lecomte, S.; Smith, W.E. Journal of Raman Spectroscopy 1999, 30, 37-44.
(16) Sanchezcortes, S.; Garciaramos, J.V.; Morcillo, G.; Tinti, A. Journal of Colloid and Interface Science 1995, 175, 358-368.
(17) Blatchford, C.G.; Campbell, J.R.; Creighton, J.A. Surface Science 1982, 120, 435-455.
(18) Jones, J.C.; McLaughlin, C.; Littlejohn, D.; Sadler, D.A.; Graham, D.; Smith, W.E. Analytical Chemistry 1999, 71, 596-601.
(19) Xu, H.X.; Aizpurua, J.; Kall, M.; Apell, P. Physical Review E 2000, 62, 4318-4324.
(20) Stockman, M.I.; Shalaev, V.M.; Moskovits, M.; Botet, R.; George, T.F. Physical Review B 1992, 46, 2821-2830.
(21) Vlckova, B.; Gu, X.J.; Moskovits, M. Journal of Physical Chemistry B 1997, 101, 1588-1593.
(22) Laserna, J.J.; Berthod, A.; Winefordner, J.D. Microchemical Journal 1988, 38, 125-136.
(23) Cabalin, L.M.; Ruperez, A.; Laserna, J.J. Analytica Chimica Acta 1996, 318, 203-210.
(24) Sheng, R.S.; Ni, F.; Cotton, T.M. Analytical Chemistry 1991, 63, 437-442.
(25) Freeman, R.D.; Hammaker, R.M.; Meloan, C.E.; Fateley, W.G. Applied Spectroscopy 1988, 42, 456-460.
(26) Taylor, G.T.; Sharma, S.K.; Mohanan, K. Applied Spectroscopy 1990, 44, 635-640.
(27) Laserna, J.J.; Berthod, A.; Winefordner, J.D. Talanta 1987, 34, 745-747.
(28) Laserna, J.J., Berthod, A., Winfordner, J.D. Michrochemical Journal 1988, 38, 125-136.
(29) Berthod, A.; Laserna, J.J.; Winefordner, J.D. Applied Spectroscopy 1987, 41, 1137-1141.
(30) Ruano, J.M.; Benoit, V.; Aitchison, J.S. and Cooper, J.M., Flame Hydrolysis Deposition of Glass on Silicon for the Integration of Optical and Microfluidic Devices, Analytical Chemistry, 2000, 72, 1093-1097.
(31) Pillai, V.N.R.; Synthesis 1980, 1-26.

## Claims

1. A method of generating *in situ* a colloid for use in detecting an analyte using SER(R)S, wherein the colloid is generated by contacting under conditions of laminar flow a first microfluidic stream of a suitable metal salt with a second microfluidic stream of a reducing agent whereby mixing of said first and second streams substantially occurs at an interface region between said first and second streams and wherein colloid is formed in the interface region and wherein the two streams are mixed in a channel of 1 µm to 500 µm across .

2. A method of detecting the presence of an analyte in a sample, comprising the steps of:
1) generating in *situ* the colloid according to claim 1, or *ex situ*;
2) admixing said said sample with said colloid, in a microfluidic system having channels of 1 µm to 500 µm across under laminar flow conditions suitable to allow any of said analyte present in the sample to adhere to said colloid; and
3) detecting the presence of any of said analyte in the sample by way of SER(R)S.

3. The method according to claim 2 wherein the sample or samples are provided by way of a further microfluidic stream(s) arranged to contact the colloid so formed.

4. The method according to any preceding claim further comprising the addition of an aggregating agent for colloid generation, said aggregating agent being introduced by way of a further microfluidic stream, or included in said first or second microfluidic stream, which is combined with said other streams, before, during, or after sample mixing.

5. The method according to any one of claims 2 to 4 wherein the analyte to be detected is selected from nucleic acids, nucleic acid analogues, proteins, peptides, amino acids, enzymes, prions, antibodies, aldehydes, amines, ketones, explosives, drugs of abuse, therapeutic agents, metabolites and environmental pollutants.

6. The method according to any of claims 2 to 5 wherein the sample is a sample of gas or liquid.

7. The method according to any one of claims 2 to 6 wherein the analyte is in a fluid or is transferred to a fluid before mixing with the colloid *in situ* or ex *situ.*

8. The method according to any of claims 2 to 7 wherein an initial sample is obtained from a source and any target analyte present in said initial sample is absorbed onto a suitable substrate, and thereafter, any target analyte is removed from the substrate by washing with a suitable solvent, so as to form the sample to be tested.

9. The method according to any one of claims 2 to 8 wherein a chromophore of a suitable wavelength to be in resonance with a laser chosen for use in said SER (R) S detection is present in the analyte or a chromophore is created by derivatisation of the analyte before analysis.

10. The method according to any one of claims 2 to 9 wherein the analyte is reacted with a reagent so as to derivatise the analyte, and wherein the reagent which is used to derivatise the analyte, provides a chromophore, provides in combination with the analyte, a chromophore and/or renders the analyte susceptible to adhering to the SER(R)S active substrate.

11. The method according to claim 10 wherein the reagent is bound to the colloid.

12. The method according to any preceding claim wherein the colloid is coated on the surface of a particle or particles.

13. The method according to claim 12 wherein said particle(s) is/are formed of silica or a polymer such as polystyrene.

14. The method according to any preceding claim wherein particles of said colloid are further modified to comprise a SER(R)S reactive agent adhered to the particle.

15. The method according to claim 14 wherein the SER (R) S reactive agent is a dye.

16. The method according to either of claims 14 or 15 wherein more than one SER(R)S reactive agent and/or dye is adhered to the particle.

17. The method according to any one of claims 14 to 16 wherein said SER(R)S reactive agent(s) and/or dye is/are provided and mixed with the colloid, by way of an additional microfluidic stream or streams converging with the colloid stream.

18. The method according to any one of claims 14 to 17 wherein appropriate selection of said SER(R)S active agent(s) and/or dye(s) allows said colloid particle(s) to generate a specific SER(R)S signal, such that in a mixture of differently labeled colloid particles, more than one SER(R)S signal can be analysed simultaneously and said colloid particles identified based on a combined SER(R)S signal, or individual signals can be discerned within a colloid mixture comprising differently labeled colloid particles.

19. The method according to any preceding claim wherein the colloid particles prepared are aggregated in a controlled manner so as to be of a reproducible size and shape and as stable as possible against self-aggregation.

20. The method according to claim 19 wherein the colloid particles are aggregated *in situ* by contacting with a suitable aggregating agent, such as acids (eg. HNO₃ or ascorbic acid), polyamines (eg. polylysine, spermine, spermidine, 1,4-diaminopiperazine, diethylenetriamine, N-(2-aminoethyl)-1,3-propanediamine, triethylenetetramine and tetraethylenepentamine) and inorganic activating ions such as Cl⁻, I⁻, Na⁺ or Mg²⁺, or aggregating means comprising electrochemical, electric, dielectric or magnetic means.

21. The method according to any preceding claim wherein the first stream comprises sodium-borohydride and the second stream comprises silver nitrate.

22. The method according to any preceding claim wherein the analyte to be detected is within a biological cell and wherein the method further comprises the steps of
a) immobilizing or retaining said cell;
b) permeabilising said cell so as to allow colloid to flow into and/or through the cell such that colloid particles are capable of binding to said analyte within said cell; and
c) detecting binding of colloid particles to said analyte by way of SER (R) S.

23. The method according to claim 22 wherein the cell is an animal cell.

24. The method according to either of claims 22 or 23 wherein the cell is permeabilised by electroporation.

25. The method according to any one of claims 2 to 24 wherein said SER(R)S signal is collected using wavelength selective mirrors, and/or gratings, holographic optical elements for scattered light detection, lenses, integrated waveguides or fibre-optic waveguides.

26. The method according to any one of claims 2 to 25 wherein the intensity of a SER(R)S signal is measured using a charge coupled device (CCD), a silicon photodiode, CMOS integrated detector(s), photomultiplier tubes arranged either singly or in series for cascade amplification of the signal, or for photon counting electronics.

27. A microfluidic device for use in detecting analytes by way of SER(R)S, the device comprising a substrate having microscale channels of 1µm to 500µm across formed therein and inlets attached to the channels for introducing a suitable metal salt, a reducing agent, a sample and optionally an aggregating agent, wherein laminar flow within the channels for carrying the suitable metal salt and the reducing agent converge so as to allow mixing of the suitable metal salt and the reducing agent and generation of a colloid at an interface between the suitable metal salt and the reducing agent; and wherein laminar flow within a channel for carrying said sample is arranged to converge with the colloid so produced such that any analyte present in the sample is capable of adhering to the colloid, thereby allowing detection by SER(R)S to be carried out.

28. The device according to claim 27 wherein the substrate is formed of silicon, glass, or polymer.

29. The device according to either of claims 27 or 28 wherein the microscale channels have dimensions in the range 10µm - 200µm across.

30. The device according to any one of claims 28 to 29 wherein the inlets for introducing the various reagents are in the form of wells or reservoirs for maintaining a suitable quantity of reagent or analyte/sample.

31. The device according to any one of claims 27 to 30 further comprising a channel for carrying said colloid with adhered analyte, such that SER (R) S detection means are capable of being focussed on said channel so as to enable SER(R)S detection to be carried out.

32. The device according to any one of claims 27 to 31 wherein said colloid forming reagents and sample are drawn through the channels by a pump, syringe drive, electrokinetics and/or electrohydrodynamics.

33. The device according to any one of claims 27 to 32 further comprising a microelectrode or microelectrodes in communication with the substrate and/or fluid

34. The device according to claim 33 wherein said microelectrodes are in the form of an array.

35. The device according to either of claims 33 or 34 wherein the microelectrode(s) can be used to further influence the interfacial stream of colloidal particles by directing the stream into a separate analytical channel, chamber or region by use of electrophoresis, dielectrophoresis or the generation of an electro-osmotic flow.

36. The device according to any one of claims 27 to 35 wherein control of the flow of the colloid is further or alternatively controlled by optical fields or by magnetism.

37. The device according to any one of claims 27 to 36 further comprising waveguides or fibres for localising the excitation or collection of light.

38. The method for detecting an analyte according to the method of claims 2 - 26 wherein the analyte has been modified to contain a tag, wherein the tagged analyte is substantially incapable of producing a SER(R)S signal, or produces a poorly distinguishable SER (R) S signal, but is capable of producing a sufficiently distinguishable SER (R) S signal when in combination, or after reaction with a SER(R)S activating agent, comprising the steps of:
a) carrying out the method according to any one of claims 2 to 26 wherein the sample comprises said tagged analyte and said tagged analyte is reacted or combined with the SER(R)S activating agent, prior to admixing, or after admixing the colloid with the tagged analyte.

39. The method according to claim 38 wherein the analyte to be tagged is an oligonucleotide, protein, peptide, or biomolecule.

40. The method according to either of claims 38 or 39 wherein the tag is a diene or dienophile and the SER(R)S activating agent is a dienophile or diene respectively.

41. The method according to claim 40 wherein the diene is a furan or butadiene residue.

42. The method according to claim 40 wherein the dienophile is a maleimide.

43. The method according to claim 42 wherein the maleimide is capable of surface absorption such as benzotriazole maleimide or benzotriazole azo dye maleimide.

## Patentansprüche

1. Verfahren zum Erzeugen *in situ* eines Kolloids zur Verwendung beim Nachweisen eines Analyts unter Verwendung von SER(R)S, wobei das Kolloid durch Inkontaktbringen unter Bedingungen laminarer Strömung eines ersten mikrofluidischen Stroms eines geeigneten Metallsalzes mit einem zweiten mikrofluidischen Strom eines Reduktionsmittels erzeugt wird, wodurch Mischen des ersten und zweiten Stromes im wesentlichen an einer Grenzflächenregion zwischen dem ersten und zweiten Strom erfolgt und wobei in der Grenzflächenregion Kolloid erzeugt wird und wobei die zwei Ströme in einem Kanal von 1 µm bis 500 µm Breite gemischt werden.

2. Verfahren zum Nachweisen der Anwesenheit eines Analyts in einer Probe, umfassend die Schritte:
1) Erzeugen des Kolloids nach Anspruch 1 *in situ* oder *ex situ:*
2) Mischen der Probe mit dem Kolloid in einem mikrofluidischen System mit Kanälen von 1 µm bis 500 µm Breite unter laminaren Strömungsbedingungen, geeignet, einem von dem Analyt, vorhanden in der Probe, zu erlauben, an dem Kolloid zu haften; und
3) Nachweisen der Anwesenheit von einem von dem Analyt in der Probe durch SER(R)S.

3. Verfahren nach Anspruch 2, wobei die Probe oder die Proben durch einen weiteren mikrofluidischen Strom (weitere mikrofluidische Ströme), angeordnet, um mit dem so erzeugten Kolloid in Kontakt zu kommen, bereitgestellt werden.

4. Verfahren nach einem vorhergehenden Anspruch, weiterhin umfassend die Zugabe eines Aggregierungsmittels zur Kolloiderzeugung, wobei das Aggregierungsmittel durch einen weiteren mikrofluidischen Strom eingeführt oder in den ersten oder zweiten mikrofluidischen Strom eingeschlossen wird, welcher mit den anderen Strömen vor, während oder nach dem Mischen der Probe vereinigt wird.

5. Verfahren nach einem der Ansprüche 2 bis 4, wobei der nachzuweisende Analyt aus Nucleinsäuren, Nucleinsäureanalogen, Proteinen, Peptiden, Aminosäuren, Enzymen, Prionen, Antikörpern, Aldehyden, Aminen, Ketonen, Explosivstoffen, Suchtmitteln, therapeutischen Mitteln, Metaboliten und Umweltschadstoffen ausgewählt ist.

6. Verfahren nach einem der Ansprüche 2 bis 5, wobei die Probe eine Probe von Gas oder Flüssigkeit ist.

7. Verfahren nach einem der Ansprüche 2 bis 6, wobei der Analyt in einem Fluid ist oder vor dem Mischen mit dem Kolloid *in situ* oder *ex situ* in ein Fluid überführt wird.

8. Verfahren nach einem der Ansprüche 2 bis 7, wobei eine anfängliche Probe aus einem Ausgangsstoff erhalten wird und ein Zielanalyt, vorhanden in der anfänglichen Probe, auf ein geeignetes Substrat absorbiert wird und danach ein Zielanalyt durch Waschen mit einem geeigneten Lösungsmittel von dem Substrat entfernt wird, um die zu testende Probe zu erzeugen.

9. Verfahren nach einem der Ansprüche 2 bis 8, wobei ein Chromophor einer geeigneten Wellenlänge, um in Resonanz mit einem Laser, ausgewählt zur Verwendung in dem SER(R)S-Nachweis, zu sein, in dem Analyt vorhanden ist oder durch Derivatisierung des Analyts vor der Analyse ein Chromophor erzeugt wird.

10. Verfahren nach einem der Ansprüche 2 bis 9, wobei der Analyt mit einem Reagenz umgesetzt wird, um den Analyten zu derivatisieren, und wobei das Reagenz, welches verwendet wird, um den Analyten zu derivatisieren, einen Chromophor bereitstellt, in Kombination mit dem Analyten einen Chromophor bereitstellt und/oder den Analyten zum Haften an dem SER(R)S-aktiven Substrat zugänglich macht.

11. Verfahren nach Anspruch 10, wobei das Reagenz an das Kolloid gebunden wird,

12. Verfahren nach einem vorhergehenden Anspruch, wobei das Kolloid auf der Oberfläche eines Teilchens oder von Teilchen aufgebracht wird.

13. Verfahren nach Anspruch 12, wobei das (die) Teilchen aus Siliciumdioxid oder einem Polymer wie beispielsweise Polystyrol erzeugt ist/sind.

14. Verfahren nach einem vorhergehenden Anspruch, wobei Teilchen des Kolloids weiter modifiziert werden, um ein SER(R)S-reaktives Mittel, haftend an dem Teilchen, zu umfassen.

15. Verfahren nach Anspruch 14, wobei das SER(R)S-reaktive Mittel ein Farbstoff ist.

16. Verfahren nach einem der Ansprüche 14 oder 15, wobei mehr als ein SER(R)S-reaktives Mittel und/oder Farbstoff an dem Teilchen hattet.

17. Verfahren nach einem der Ansprüche 14 bis 16, wobei das SER(R)S-reaktive Mittel (die SER(R)S-reaktiven Mittel) und/oder der Farbstoff durch einen zusätzlichen mikrofluidischen Strom oder Ströme, zusammenlaufend mit dem Kolloidstrom, bereitgestellt und mit dem Kolloid gemischt wird/werden.

18. Verfahren nach einem der Ansprüche 14 bis 17, wobei geeignete Auswahl des SER(R)S-aktiven Mittels (der SER(R)S-aktiven) Mittel) und/oder des Farbstoffs (der Farbstoffe) dem (den) kolloiden Teilchen erlaubt, ein spezifisches SER(R)S-Signal zu erzeugen, derart, daß in einem Gemisch von unterschiedlich **gekennzeichnet**en kolloiden Teilchen mehr als ein SER(R)S-Signal gleichzeitig analysiert werden kann und die identifizierten kolloiden Teilchen, basierend auf einem kombinierten SER(R)S-Signal oder individuellen Signalen innerhalb eines kolloiden Gemisches, umfassend unterschiedlich **gekennzeichnet**e kolloide Teilchen, wahrgenommen werden können.

19. Verfahren nach einem vorhergehenden Anspruch, wobei die hergestellten kolloiden Teilchen in einer gesteuerten Weise aggregiert werden, um eine reproduzierbare Größe und Gestalt zu haben und so stabil wie möglich gegen Selbstaggregation zu sein.

20. Verfahren nach Anspruch 19, wobei die kolloiden Teilchen *in situ* durch Inkontaktbringen mit einem geeigneten Aggregierungsmittel, wie beispielsweise Säuren (z.B. HNO₃ oder Ascorbinsäure), Polyamine (z.B. Polylysin, Spermin, Spermidin, 1,4-Diaminopiperazin, Diethylentriamin, N-(2-Aminoethyl)-1,3-proparidiamin, Triethylentetramin und Tetraethylenpentamin) und anorganische aktivierende Ionen, wie beispielsweise Cl⁻, r, Na⁺ oder Mg²⁺, oder Aggregierungsmittel, umfassend elektrochemische, elektrische, dielektrische oder magnetische Mittel, aggregiert werden.

21. Verfahren nach einem vorhergehenden Anspruch, wobei der erste Strom Natriumborhydrid umfaßt und der zweite Strom Silbernitrat umfaßt.

22. Verfahren nach einem vorhergehenden Anspruch, wobei der nachzuweisende Analyt innerhalb einer biologischen Zelle ist und wobei das Verfahren weiterhin die Schritte umfaßt
a) Immobilisieren oder Zurückhalten der Zelle;
b) Permeabilisieren der Zelle, um dem Kolloid zu erlauben, in und/oder durch die Zelle zu fließen, derart, daß kolloide Teilchen imstande sind, an den Analyten innerhalb der Zelle zu binden; und
c) Nachweisen der Bindung der kolloiden Teilchen an den Analyten durch SER(R)S.

23. Verfahren nach Anspruch 22, wobei die Zelle eine tierische Zelle ist.

24. Verfahren nach einem der Ansprüche 22 oder 23, wobei die Zelle durch Elektroporation permeabilisiert wird.

25. Verfahren nach einem der Ansprüche 2 bis 24, wobei das SER(R)S-Signal unter Verwendung von Wellenlängen-selektiven Spiegeln und/oder Gittern, holographischen optischen Elementen zum Streulichtnachweis, Linsen, integrierten Wellenleitern oder faseroptischen Wellenleitern gesammelt wird.

26. Verfahren nach einem der Ansprüche 2 bis 25, wobei die Intensität eines SER(R)S-Signals unter Verwendung eines ladungsgekoppelten Bauelements (CCD), einer Siliciumphotodiode, eines integrierten CMOS-Detektors (von integrierten CMOS-Detektoren), von Photomultiplierröhren, angeordnet entweder einzeln oder hintereinander zur Kaskadenverstärkung des Signals oder zur Photonenzählungselektronik, gemessen wird.

27. Mikrofluidische Vorrichtung zur Verwendung beim Nachweisen von Analyten durch SER(R)S, wobei die Vorrichtung ein Substrat mit darin erzeugten Mikrokanälen von 1 µm bis 500 µm Breite und mit den Kanälen verbundene Einlässe zum Einführen eines geeigneten Metallsalzes, eines Reduktionsmittels, einer Probe und gegebenenfalls eines Aggregierungsmittels umfaßt, wobei die laminare Strömung innerhalb der Kanäle zum Tragen des geeigneten Metallsalzes und des Reduktionsmittels zusammenläuft, um Mischen des geeigneten Metallsalzes und des Reduktionsmittels und Erzeugung eines Kolloids an einer Grenzfläche zwischen dem geeigneten Metallsalz und dem Reduktionsmittel zu erlauben; und wobei die laminare Strömung innerhalb eines Kanals zum Tragen der Probe angeordnet wird, um mit dem so hergestellten Kolloid zusammenzulaufen, derart, daß ein in der Probe vorhandener Analyt imstande ist, an dem Kolloid zu haften, wodurch erlaubt wird, daß der Nachweis durch SER(R)S ausgeführt wird.

28. Vorrichtung nach Anspruch 27, wobei das Substrat aus Silikon, Glas oder Polymer erzeugt wird.

29. Vorrichtung nach einem der Ansprüche 27 oder 28, wobei die Mikrokanäle Dimensionen in dem Bereich 10 µm - 200 µm Breite haben.

30. Vorrichtung nach einem der Ansprüche 28 bis 29, wobei die Einlässe zum Einführen der verschiedenartigen Reagenzien in der Form von Vertiefungen oder Reservoiren zum Aufrechterhalten einer geeigneten Quantität von Reagenz oder Analyt/Probe sind.

31. Vorrichtung nach einem der Ansprüche 27 bis 30, weiterhin umfassend einen Kanal zum Tragen des Kolloids mit anhaftendem Analyt, derart, daß SER(R)S-Nachweismittel imstande sind, auf den Kanal fokussiert zu werden, um zu ermöglichen, daß SER(R)S-Nachweis ausgeführt wird.

32. Vorrichtung nach einem der Ansprüche 27 bis 31, wobei die Kolloid erzeugenden Reagenzien und die Probe mit einer Pumpe, einen Spritzenantrieb, Elektrokinetik und/oder Elektrodynamik durch die Kanäle gezogen werden.

33. Vorrichtung nach einem der Anspruche 27 bis 32, weiterhin umfassend eine Mikroelektrode oder Mikroelektroden in Kommunikation mit dem Substrat und/oder Fluid.

34. Vorrichtung nach Anspruch 33, wobei die Mikroelektroden in der Form einer Anordnung sind.

35. Vorrichtung nach einem der Ansprüche 33 oder 34, wobei die Mikroelektrode(n) verwendet werden kann (können), um den Grenzflächenstrom kolloidaler Teilchen durch Richten des Stromes in einen gesonderten analytischen Kanal, eine Kammer oder eine Region durch Verwendung von Elektrophorese, Dielektrophorese oder die Erzeugung eines elektroosmotischen Flusses weiter zu beeinflussen.

36. Vorrichtung nach einem der Ansprüche 27 bis 35, wobei Steuerung des Flusses des Kolloids weiterhin oder alternativ durch optische Felder oder durch Magnetismus gesteuert wird.

37. Vorrichtung nach einem der Ansprüche 27 bis 36, weiterhin umfassend Wellenleiter oder Fasern zum Lokalisieren der Anregung oder Sammlung von Licht.

38. Verfahren zum Nachweisen eines Analyten nach dem Verfahren der Ansprüche 2-26, wobei der Analyt modifiziert worden ist, um eine Markierung zu enthalten, wobei der markierte Analyt im wesentlichen nicht imstande ist, ein SER(R)S-Signal zu erzeugen, oder ein schlecht unterscheidbares SER(R)S-Signal erzeugt, aber imstande ist, ein ausreichend unterscheidbares SER(R)S-Signal zu erzeugen, wenn in Kombination oder nach Reaktion mit einem SER(R)S-Aktivierungsmittel, umfassend die Schritte:
a) Ausführen des Verfahrens nach einem der Ansprüche 2 bis 26, wobei die Probe den markierten Analyten umfaßt und der markierte Analyt vor dem Mischen oder nach dem Mischen des Kolloids mit dem markierten Analyten mit dem SER(R)S-Aktivierungsmittel umgesetzt oder vereinigt wird.

39. Verfahren nach Anspruch 38, wobei der zu markierende Analyt ein Oligonucleotid, Protein, Peptid oder Biomolekül ist.

40. Verfahren nach einem der Ansprüche 38 oder 39, wobei die Markierung ein Dien oder Dienophil ist und das SER(R)S-Aktivierungsmittel ein Dienophil bzw. Dien ist.

41. Verfahren nach Anspruch 40, wobei das Dien ein Furan- oder Butadienrest ist.

42. Verfahren nach Anspruch 40, wobei das Dienophil ein Maleimid ist.

43. Verfahren nach Anspruch 42, wobei das Maleimid zur Oberflächenabsorption imstande ist, wie beispielsweise Benzotriazol-Maleimid oder Benzotriazol-Azofarbstoff Maleimid.

## Revendications

1. Procédé de formation *in situ* d'un colloïde pour l'utilisation dans la détection d'un analyte en utilisant une SER(R)S, dans lequel le colloïde est formé en mettant en contact, dans des conditions de flux laminaire, un premier courant microfluidique d'un sel de métal approprié avec un second courant microfluidique d'un agent réducteur de sorte que le mélange desdits premier et second courants se produit essentiellement à une région d'interface entre lesdits premier et second courants, et dans lequel le colloïde se forme dans la région d'interface et dans lequel les deux courants sont mélangés dans un canal de 1 µm à 500µm de diamètre.

2. Procédé de détection de la présence d'un analyte dans un échantillon, comprenant les étapes consistant:
1) à former le colloïde *in situ* d'après la revendication 1, ou *ex situ;*
2) à mélanger ledit échantillon avec ledit colloïde, dans un système microfluidique ayant des canaux de 1µm à 500µm de diamètre, dans des conditions de flux laminaire appropriées pour permettre à l'un quelconque dudit analyte présent dans l'échantillon d'adhérer audit colloïde; et
3) à détecter la présence de l'un quelconque dudit analyte dans l'échantillon au moyen de SER(R)S.

3. Procédé selon la revendication 2, dans lequel l'échantillon ou les échantillons sont fournis au moyen d'un (de) courant(s) microfluidique(s) supplémentaire(s) agencé(s) pour venir au contact du colloïde ainsi formé.

4. Procédé selon l'une quelconque des revendications précédentes, comprenant en outre l'addition d'un agent d'agrégation pour la formation du colloïde, ledit agent d'agrégation étant introduit au moyen d'un courant microfluidique supplémentaire, ou inclus dans ledit premier ou second courant microfluidique, qui est combiné avec lesdits autres courants, avant, pendant ou après le mélange de l'échantillon.

5. Procédé selon l'une quelconque des revendications 2 à 4, dans lequel l'analyte à détecter est choisi parmi les acides nucléiques, les analogues d'acides nucléiques, les protéines, les peptides, les acides aminés, les enzymes, les prions, les anticorps, les aldéhydes, les amines, les cétones, les explosifs, les substances toxicomanogènes, les agents thérapeutiques, les métabolites et les polluants environnementaux.

6. Procédé selon l'une quelconque des revendications 2 à 5, dans lequel l'échantillon est un échantillon de gaz ou de liquide.

7. Procédé selon l'une quelconque des revendications 2 à 6, dans lequel l'analyte est dans un fluide ou est transféré à un fluide avant le mélange avec le colloïde *in situ* ou *ex situ*.

8. Procédé selon l'une quelconque des revendications 2 à 7, dans lequel un échantillon initial est obtenu à partir d'une source et tout analyte cible présent dans ledit échantillon initial est absorbé sur un substrat approprié, puis tout analyte cible est enlevé du substrat par lavage avec un solvant approprié, de façon à former l'échantillon à tester.

9. Procédé selon l'une quelconque des revendications 2 à 8, dans lequel un chromophore d'une longueur d'onde appropriée pour être en résonance avec un laser choisi pour l'utilisation dans ladite détection SER(R)S est présent dans l'analyte ou un chromophore est créé par transformation de l'analyte avant l'analyse.

10. Procédé selon l'une quelconque des revendications 2 à 9, dans lequel l'analyte est mis à réagir avec un réactif de façon à transformer l'analyte, et dans lequel le réactif qui est utilisé pour transformer l'analyte fournit un chromophore, fournit, en combinaison avec l'analyte, un chromophore et/ou rend l'analyte susceptible d'adhérer au substrat actif SER(R)S.

11. Procédé selon la revendication 10, dans lequel le réactif est lié au colloïde.

12. Procédé selon l'une quelconque des revendications précédentes, dans lequel le colloïde est appliqué sur la surface d'une particule ou de particules.

13. Procédé selon la revendication 12, dans lequel ladite (lesdites) particule(s) est (sont) formée(s) de silice ou d'un polymère tel qu'un polystyrène.

14. Procédé selon l'une quelconque des revendications précédentes, dans lequel les particules dudit colloïde sont en outre modifiées pour comprendre un agent réactif SER(R)S collé à la particule.

15. Procédé selon la revendication 14, dans lequel l'agent réactif SER(R)S est un colorant.

16. Procédé selon l'une quelconque des revendications 14 ou 15, dans lequel plus d'un agent réactif SER(R)S et/ou colorant est collé à la particule.

17. Procédé selon l'une quelconque des revendications 14 à 16, dans lequel ledit (lesdits) agent(s) réactif(s) SER(12)S et/ou le colorant est (sont) fourni(s) et mélangé(s) avec le colloïde, au moyen d'un courant ou de courants microfluidique(s) supplémentaire(s) convergeant avec le courant de colloïde.

18. Procédé selon l'une quelconque des revendications 14 à 17, dans lequel un choix approprié dudit (desdits) agent(s) actif(s) SER(R)S et/ou colorant(s) permet à ladite (auxdites) particule(s) colloïdale(s) de générer un signal SER(R)S spécifique, de sorte que dans un mélange de particules colloïdales marquées différemment, plus d'un signal SER(R)S peut être analysé simultanément et lesdites particules colloïdales identifiées sur la base d'un signal SER(R)S combiné, ou des signaux individuels peuvent être discernés à l'intérieur d'un mélange colloïdal comprenant des particules colloïdales marquées différemment.

19. Procédé selon l'une quelconque des revendications précédentes, dans lequel les particules colloïdales préparées sont agrégées d'une manière contrôlée de façon à être d'une dimension et d'une forme reproductibles et aussi stables que possible contre une auto-agrégation.

20. Procédé selon la revendication 19, dans lequel les particules colloïdales sont agrégées *in situ* par mise en contact avec un agent d'agrégation approprié, tel que des acides (par exemple, HNO₃ ou acide ascorbique), des polyamines (par exemple, polylysine, spermine, spermidine, 1,4-diaminopipérazine, diéthylènetriamine, N-(2-aminoéthyl)-1,3-propanediamine, triéthylènetétramine et tétraéthylènepentamine) et des ions activateurs inorganiques tels que Cl⁻, I⁻, Na⁺ ou Mg²⁺, ou des moyens d'agrégation comprenant des moyens électrochimiques, électriques, diélectriques ou magnétiques.

21. Procédé selon l'une quelconque des revendications précédentes, dans lequel le premier courant comprend du borohydrure de sodium et le second courant comprend du nitrate d'argent.

22. Procédé selon l'une quelconque des revendications précédentes, dans lequel l'analyte à détecter est à l'intérieur d'une cellule biologique et dans lequel le procédé comprend en outre les étapes consistant
a) à immobiliser ou retenir ladite cellule;
b) à perméabiliser ladite cellule de façon à permettre au colloïde de s'écouler dans et/ou à travers la cellule de sorte que les particules colloïdales sont aptes à se lier audit analyte à l'intérieur de ladite cellule; et
c) à détecter la liaison des particules colloïdales audit analyte au moyen de SER(R)S.

23. Procédé selon la revendication 22, dans lequel la cellule est une cellule animale.

24. Procédé selon l'une quelconque des revendications 22 ou 23, dans lequel la cellule est perméabilisée par électroporation.

25. Procédé selon l'une quelconque des revendications 2 à 24, dans lequel ledit signal SER(R)S est recueilli en utilisant des miroirs sélectifs à longueur d'onde, et/ou des grilles, des éléments optiques holographiques pour détection de lumière dispersée, des lentilles, des guides d'ondes intégrés ou des guides d'ondes à fibres optiques.

26. Procédé selon l'une quelconque des revendications 2 à 25, dans lequel l'intensité d'un signal SER(R)S est mesurée en utilisant un dispositif à couplage de charge (CCD), une photodiode au silicium, un (des) détecteur(s) à CMOS intégré, des tubes photomultiplicateurs agencés soit isolément soit en série pour l'amplification en cascade du signal, ou pour des systèmes électroniques de comptage de photons.

27. Dispositif microfluidique pour l'utilisation dans la détection d'analytes au moyen de SER(R)S, le dispositif comprenant un substrat ayant des canaux à petite échelle de 1 µm à 500µm de diamètre formés dans celui-ci et des entrées attachées aux canaux pour introduire un sel de métal approprié, un agent réducteur, un échantillon et facultativement un agent d'agrégation, dans lequel le flux laminaire à l'intérieur des canaux pour transporter le sel de métal approprié et l'agent réducteur convergent de façon à permettre le mélange du sel de métal approprié et de l'agent réducteur et la formation d'un colloïde à une interface entre le sel de métal approprié et l'agent réducteur; et dans lequel le flux laminaire à l'intérieur d'un canal pour transporter ledit échantillon est agencé pour converger avec le colloïde ainsi produit de sorte que tout analyte présent dans l'échantillon est apte à adhérer au colloïde, ce qui permet à la détection par SER(R)S d'être effectuée.

28. Dispositif selon la revendication 27, dans lequel le substrat est formé de silicium, de verre ou de polymère.

29. Dispositif selon l'une quelconque des revendications 27 ou 28, dans lequel les canaux à petite échelle ont des dimensions dans la gamme de 10µm à 200µm de diamètre.

30. Dispositif selon l'une quelconque des revendications 28 à 29, dans lequel les entrées pour introduire les divers réactifs sont sous la forme de puits ou de réservoirs pour maintenir une quantité appropriée de réactif ou d'analyte/échantillon.

31. Dispositif selon l'une quelconque des revendications 27 à 30, comprenant en outre un canal pour transporter ledit colloïde avec l'analyte adhéré, de sorte que les moyens de détection SER(R)S sont aptes à être focalisés sur ledit canal de façon à permettre à la détection SER(R)S d'être effectuée.

32. Dispositif selon l'une quelconque des revendications 27 à 31, dans lequel lesdites réactifs formant un colloïde et l'échantillon sont attirés à travers les canaux par une pompe, un entraînement de seringue, des dispositifs électrocinétiques et/ou électrohydrodynamiques.

33. Dispositif selon l'une quelconque des revendications 27 à 32, comprenant en outre une microélectrode ou des microélectrodes en communication avec le substrat et/ou un fluide.

34. Dispositif selon la revendication 33, dans lequel lesdites microélectrodes sont sous la forme d'un alignement.

35. Dispositif selon l'une quelconque des revendications 33 ou 34, dans lequel la (les) microélectrode(s) peut (peuvent) être utilisée(s) pour influencer plus encore le courant interfacial des particules colloïdales en dirigeant le courant jusque dans un canal, une chambre ou une région analytiques séparés, en utilisant une électrophorèse, une diélectrophorèse ou la formation d'un flux électro-osmotique.

36. Dispositif selon l'une quelconque des revendications 27 à 35, dans lequel le contrôle du flux du colloïde est en outre ou en variante contrôlé par des champs optiques ou par magnétisme.

37. Dispositif selon l'une quelconque des revendications 27 à 36, comprenant en outre des guides d'ondes ou des fibres pour localiser l'excitation ou la collecte de lumière.

38. Procédé pour détecter un analyte selon le procédé des revendications 2 à 26, dans lequel l'analyte est modifié pour contenir un marqueur, dans lequel l'analyte marqué est pratiquement incapable de produire un signal SER(R)S, ou produit un signal SER(R)S peu distinguable, mais est capable de produire un signal SER(R)S suffisamment distinguable lorsqu'il est combiné, ou après réaction avec un agent activant SER(R)S, comprenant les étapes consistant :
a) à mettre en oeuvre le procédé selon l'une quelconque des revendications 2 à 26, dans lequel l'échantillon comprend ledit analyte marqué et ledit analyte marqué est mis à réagir ou combiné avec l'agent activant SER(R)S, avant le mélange, ou après le mélange du colloïde avec l'analyte marqué.

39. Procédé selon la revendication 38, dans lequel l'analyte à marquer est un oligonucléotide, une protéine, un peptide ou une biomolécule.

40. Procédé selon l'une quelconque des revendications 38 ou 39, dans lequel le marqueur est un diène ou un diénophile et l'agent activant SER(R)S est un diénophile ou un diène respectivement.

41. Procédé selon la revendication 40, dans lequel le diène est un reste furanne ou butadiène.

42. Procédé selon la revendication 40, dans lequel le diénophile est un maléimide.

43. Procédé selon la revendication 42, dans lequel le maléimide est apte à une absorption de surface, tel que benzotriazole maléimide ou benzotriazole colorant azoïque maléimide.
